# EUROPEAN PATENT SPECIFICATION

(11) **EP 1 736 484 B1**
(45) Date of publication and mention of the grant of the patent: **06.10.2010**
(21) Application number: 05737919.0
(22) Date of filing: 29.03.2005
(51) Int. Cl.: C07K 16/00

(54) **AN GENE ENGINEERING RECOMBINANT ANTI-CEA, ANTI-CD3 AND ANTI-CD28 SINGLE-CHAIN TRI-SPECIFIC ANTIBODY**
EIN DURCH GENETISCHE MANIPULATION ERZEUGTER, REKOMBINANTER TRISPEZIFISCHER ANTI-CEA-, ANTI-CD3- UND ANTI-CD28-ANTIKÖRPER MIT EINER KETTE
ANTICORPS TRISPECIFIQUE MONOCATENAIRE RECOMBINE ANTI-CEA, ANTI-CD3 ET ANTI-CD28, MIS AU POINT GENETIQUEMENT

(30) Priority: 01.04.2004 CN 200410032158
(43) Date of publication of application: 27.12.2006
(73) Proprietor: Beijing ABT Genetic Engineering Technology Co., Ltd., Beijing 102206 (CN); Dongguan Haofa Biotechnology Developmental Co. Ltd, Dongguan, Guangdong 523980 (CN)
(72) Inventor: WANG, Xiangbin, Beijing 102206 (CN); HUANG, Hualiang, Beijing 102206 (CN); ZHAO, Baofeng, Beijing 102206 (CN); ZHAO, Qi, Beijing 102206 (CN); PIAO, Jinhua, Beijing 102206 (CN); LIN, Qing, Beijing 102206 (CN)
(74) Representative: Viering, Hans-Martin
(86) International application number: PCT/CN2005/000408
(87) International publication number: WO 2005/095456

(56) References cited:
- EP-A1- 1 378 520
- CN-A- 1 380 341
- SONG LI-PING ET AL: "A NEW MODEL OF TRISPECIFIC ANTIBODY WITH CYTOTOXICITY AGAINST TUMOR CELLS" SHENGWU HUAXUE YU SHENGWU WULI XUEBAO - ACTA BIOCHIMICA ET BIOPHYSICA SINICA, SHANGHAI KEXUE JISHU CHUBANSHE, SHANGHAI,, CN, vol. 35, no. 6, June 2003 (2003-06), pages 503-510, XP002388106 ISSN: 0582-9879
- HOLLIGER P ET AL: "Carcinoembryonic antigen (CEA)-specific T-cell activation in colon carcinoma induces by anti-CD3*anti CEA bispecific diabodies and B7*antiCEA bispecific fusion proteins" CANCER RESEARCH, vol. 59, 1999, pages 2909-2916, XP002477702
- SONG L. ET AL: 'A New Model of Trispecific Antibody with Cytotyxicity against Tumor Cells.' ACTA BIOCHIMICA ET BIOPHYSICA SINICA. vol. 35, no. 6, June 2003, pages 503 - 510, XP002388106
- KOGA H. ET AL: 'Mouse-human Chimeric Monoclonal Antibody to Carcinoembryonic Antigen (CEA): in Vitro and in Vivo Activities.' HYBRIDOMA. vol. 9, no. 1, 1990, pages 43 - 48, XP008074735

### SEQUENCE LISTING

<110> BEIJIKG ABT GENETIC ENGINEERING TECHNOLOGY CO.. LTD
   DONGGUAN HAOFA BIOTECHNOLOGY DEVELOPMENTAL Co., LTD.
<120> Linear single chain recombinant anti-CEA/CD3/CD28 trispecific antibody
<130> 1040179
<160> 52
<170> Patent In version 3.1
<210> 1
   <211> 251
   <212> PRT
   <213> Artificial
<100> 1
<210> 2
   <211> 250
   <212> PRT
   <213> Artificial
<400> 2
   Glu Val Lys Leu Val Glu Ser Gly Pro Glu Leu Val Lys Pro Gly Ala
   1 5 10 15
   Ser Met Lys Ile Ser Cys Lys Ala Ser Gly Tyr Ser Phe Thr Gly Tyr
   20 25 30
   Thr Met Asn Trp Val Lys Gln Ser His Gly Lys Asn Leu Glu Trp Met
   35 40 45
   Gly Leu Ile Asn Pro Tyr Lys Gly Val Ser Thr Tyr Asn Gln Lys Phe
   50 55 60
   Lys Asp Lys Ala Thr Leu Thr Val Asp Lys Ser Ser Ser Thr Ala Tyr
   65 70 75 80
   Met Glu Leu Leu Ser Leu Thr Ser Glu Asp Ser Ala Val Tyr Tyr Cys
   85 90 95
   Ala Arg Ser Gly Tyr Tyr Gly Asp Ser Asp Trp Tyr Phe Asp Val Trp
   100 105 110
   Gly Ala Gly Thr Ser Val Thr Val Ser Ser Thr Ser Gly Gly Gly Gly
   115 120 125
   Ser Gly Gly Gly Gly Ser Gly Gly Gly Gly Ser Ser Arg Asp Ile Gln
   130 135 140
   Met Thr Gln Thr Thr Ser Ser Leu Ser Ala Ser Leu Gly Asp Arg Val
   145 150 155 160
   Thr Ile Ser Cys Arg Ala Ser Gln Asp Ile Arg Asn Tyr Leu Asn Trp
   165 170 175
   Tyr Gln Gln Lys Pro Asp Gly Thr Val Lys Leu Leu Ile Tyr Tyr Thr
   180 185 190
   Ser Arg Leu His Ser Gly Val Pro Ser Lys Phe Ser Gly Ser Gly Ser
   195 200 205
   Gly Thr Asp Tyr Ser Leu Thr Ile Ser Asn Leu Glu Gln Glu Asp Ile
   210 215 220
   Ala Thr Tyr Phe Cys Gln Gln Gly Asn Thr Leu Pro Trp Thr Phe Ala
   225 230 235 240
   Gly Gly Thr Lys Leu Glu Leu Lys Arg Ala
   245 250
<210> 3
   <211> 2103
   <212> DNA
   <213> Artificial
<400> 3
   atgggtctcg agcaggtgca gctgcagcag agcggtgcgg aactgatgaa accgggcgcg      60
   agcgtgaaaa tcagctgcaa agcgaccggc tataccttca gcgattattg gatcgaatgg     120
   gtgaaacagc gtccgggtca cggcctggaa tggatcggtg aaatcctgcc gggcagcggc     180
   cgtaccgact acaacgaacg tttcaaaggc aaagcgacct tcaccggcga cgtttctagc     240
   aacaccgcgt atatgaaact gtctagcctg accagcgaag atagcgcggt gtattactgc     300
   gcgaccggca ccaccccgtt cggttactgg ggtcagggca ccctggttac cgtttccgcg     360
   actagtaccc cgagccataa cagccatcag gtgccgagcg cgggcggccc gaccgcgaac     420
   agcggctcta gagacatcgt gctgacccag agcccggcga gcctggcggt gtctctgggt     480
   cagcgtgcga ccatctcctg ccgtgcttcc cagtccgttt ccacctcctc ctacacctac     540
   atgcactggt atcagcagaa accgggtcag ccgccgaaac tgctgatcaa atatgcgagc     600
   aacctggaat ctggtgtgcc ggcgcgtttc agcggttctg gcagcggcac cgacttcacc     660
   ctgaacatcc acccggtgga agaagaagat accgcgtatt actattgcca gcactcttgg     720
   gaaatcccgc gtaccttcgg tggcggcacc aaactggaaa tcaaagaatt caacagcacg     780
   taccgggttg taagcgtcct caccgtactg caccaggact ggctgaatgg caaggaatac     840
   aaatgcaaga gtactgaggt gaagctggtg gagtctggac ctgagctggt gaagcctgga     900
   gcttcaatga agatatcctg caaggcttct ggttactcat tcactggcta caccatgaac     960
   tgggtgaagc agagtcatgg aaagaacctt gagtggatgg gacttattaa tccttacaaa    1020
   ggtgttagta cctacaacca gaagttcaag gacaaggcca cattaactgt agacaagtca    1080
   tccagcacag cctacatgga actcctcagt ctgacatctg aggactctgc agtctattac    1140
   tgtgcaagat cggggtacta cggtgatagt gactggtact tcgatgtctg gggcgcagga   1200
   acctcagtca ctgtctcctc aactagtggt ggtggtggtt ctggtggtgg tggttctggt   1260
   ggtggtggtt cttctagaga catccagatg acccagacca catcctccct gtctgcctct   1320
   ctgggagaca gagtcaccat cagttgcagg gcaagtcagg acattagaaa ttatttaaac   1380
   tggtatcaac agaaaccaga tggaactgtt aaactcctga tctactacac atcaagatta   1440
   cactcaggag tcccatcaaa gttcagtggc agtgggtctg gaacagatta ttctctcacc   1500
   attagcaacc tggagcaaga ggatattgcc acttactttt gccaacaggg taatacgctt   1560
   ccgtggacgt tcgctggagg caccaaactg gaactgaagc gcgctgtcga cttccagaat   1620
   gcgctgctgg ttcgttacac caagaaagta ccccaagtgt caactccaac tcctgtagag   1680
   gtctcacata tgcaggtaca gctacaggaa tctggtccgg gtctggtaaa accgtctcag   1740
   accctgtctc tgacctgtac cgtatctggt ttctctctgt ctgactatgg tgttcattgg   1800
   gtacgtcagc cgccaggtaa aggtctggaa tgtctgggtg taatatgggg tggaggcacg   1860
   aattataatt cggctctcat gtccagacgt gtaacctctt ccgacgatac ctctaaaaat   1920
   cagttctctc tgaaactgtc ttccgtagac accgctgtat actattgtgc tcgttcctat   1980
   tactattcta tggactactg gggtcagggc accctggtaa ccgtatcttc cggtaccgaa   2040
   caaaaactca tctcagaaga ggatctgaat ggggccgcac atcatcatca ccatcacgag   2100
   caa                                                                 2103
<210> 4
   <211> 701
   <212> PRT
   <213> Artificial
<400> 4
   Met Gly Leu Glu Gln Val Gln Leu Gln Gln Ser Gly Ala Glu Leu Met
   1               5                   10                  15
   Lys Pro Gly Ala Ser Val Lys Ile Ser Cys Lys Ala Thr Gly Tyr Thr
               20                  25                  30
   Phe Ser Asp Tyr Trp Ile Glu Trp Val Lys Gln Arg Pro Gly His Gly
           35                  40                  45
   Leu Glu Trp Ile Gly Glu Ile Leu Pro Gly Ser Gly Arg Thr Asp Tyr
       50                  55                  60
   Asn Glu Arg Phe Lys Gly Lys Ala Thr Phe Thr Gly Asp Val Ser Ser
   65                  70                  75                  80
   Asn Thr Ala Tyr Met Lys Leu Ser Ser Leu Thr Ser Glu Asp Ser Ala
                   85                  90                  95
   Val Tyr Tyr Cys Ala Thr Gly Thr Thr Pro Phe Gly Tyr Trp Gly Gln
               100                 105                 110
   Gly Thr Leu Val Thr Val Ser Ala Thr Ser Thr Pro Ser His Asn Ser
   115 120 125
   His Gln Val Pro Ser Ala Gly Gly Pro Thr Ala Asn Ser Gly Ser Arg
   130 135 140
   Asp Ile Val Leu Thr Gln Ser Pro Ala Ser Leu Ala Val Ser Leu Gly
   145 150 155 160
   Gln Arg Ala Thr Ile Ser Cys Arg Ala Ser Gln Ser Val Ser Thr Ser
   165 170 175
   Ser Tyr Thr Tyr Met His Trp Tyr Gln Gln Lys Pro Gly Gln Pro Pro
   180 185 190
   Lys Leu Leu Ile Lys Tyr Ala Ser Asn Leu Glu Ser Gly Val Pro Ala
   195 200 205
   Arg Phe Ser Gly Ser Gly Ser Gly Thr Asp Phe Thr Leu Asn Ile His
   210 215 220
   Pro Val Glu Glu Glu Asp Thr Ala Tyr Tyr Tyr Cys Gln His Ser Trp
   225 230 235 240
   Glu Ile Pro Arg Thr Phe Gly Gly Gly Thr Lys Leu Glu Ile Lys Glu
   245 250 255
   Phe Asn Ser Thr Tyr Arg Val Val Ser Val Leu Thr Val Leu His Gln
   260 265 270
   Asp Trp Leu Asn Gly Lys Glu Tyr Lys Cys Lys Ser Thr Glu Val Lys
   275 280 285
   Leu Val Glu Ser Gly Pro Glu Leu Val Lys Pro Gly Ala Ser Met Lys
   290 295 300
   Ile Ser Cys Lys Ala Ser Gly Tyr Ser Phe Thr Gly Tyr Thr Met Asn
   305 310 315 320
   Trp Val Lys Gln Ser His Gly Lys Asn Leu Glu Trp Met Gly Leu Ile
   325 330 335
   Asn Pro Tyr Lys Gly Val Ser Thr Tyr Asn Gln Lys Phe Lys Asp Lys
   340 345 350
   Ala Thr Leu Thr Val Asp Lys Ser Ser Ser Thr Ala Tyr Met Glu Leu
   355 360 365
   Leu Ser Leu Thr Ser Glu Asp Ser Ala Val Tyr Tyr Cys Ala Arg Ser
   370 375 380
   Gly Tyr Tyr Gly Asp Ser Asp Trp Tyr Phe Asp Val Trp Gly Ala Gly
   385 390 395 400
   Thr Ser Val Thr Val Ser Ser Thr Ser Gly Gly Gly Gly Ser Gly Gly
   405 410 415
   Gly Gly Ser Gly Gly Gly Gly Ser Ser Arg Asp Ile Gln Met Thr Gln
   420 425 430
   Thr Thr Ser Ser Leu Ser Ala Ser Leu Gly Asp Arg Val Thr Ile Ser
   435 440 445
   Cys Arg Ala Ser Gln Asp Ile Arg Asn Tyr Leu Asn Trp Tyr Gln Gln
   450 455 460
   Lys Pro Asp Gly Thr Val Lys Leu Leu Ile Tyr Tyr Thr Ser Arg Leu
   465 470 475 480
   His Ser Gly Val Pro Ser Lys Phe Ser Gly Ser Gly Ser Gly Thr Asp
   485 490 495
   Tyr Ser Leu Thr Ile Ser Asn Leu Glu Gln Glu Asp Ile Ala Thr Tyr
   500 505 510
   Phe Cys Gln Gln Gly Asn Thr Leu Pro Trp Thr Phe Ala Gly Gly Thr
   515 520 525
   Lys Leu Glu Leu Lys Arg Ala Val Asp Phe Gln Asn Ala Leu Leu Val
   530 535 540
   Arg Tyr Thr Lys Lys Val Pro Gln Val Ser Thr Pro Thr Pro Val Glu
   545 550 555 560
   Val Ser His Met Gln Val Gln Leu Gln Glu Ser Gly Pro Gly Leu Val
   565 570 575
   Lys Pro Ser Gln Thr Leu Ser Leu Thr Cys Thr Val Ser Gly Phe Ser
   580 585 590
   Leu Ser Asp Tyr Gly Val His Trp Val Arg Gln Pro Pro Gly Lys Gly
   595 600 605
   Leu Glu Cys Leu Gly Val Ile Trp Gly Gly Gly Thr Asn Tyr Asn Ser
   610 615 620
   Ala Leu Met Ser Arg Arg Val Thr Ser Ser Asp Asp Thr Ser Lys Asn
   625 630 635 640
   Gln Phe Ser Leu Lys Leu Ser Ser Val Asp Thr Ala Val Tyr Tyr Cys
   645 650 655
   Ala Arg Ser Tyr Tyr Tyr Ser Met Asp Tyr Trp Gly Gln Gly Thr Leu
   660 665 670
   Val Thr Val Ser Ser Gly Thr Glu Gln Lys Leu Ile Ser Glu Glu Asp
   675 680 685
   Leu Asn Gly Ala Ala His His His His His His Glu Gln
   690 695 700
<210> 5
   <211> 18
   <212> DNA
   <213> Artificial
<400> 5
   tataccatgg gtctcgag 18
<210> 6
   <211> 59
   <212> DNA
   <213> Artificial
<400> 6
   tataccatgg gtctcgagat gtacccgcgc ggtaacacta gtgaattcaa cagcacgta 59
<210> 7
   <211> 59
   <212> DNA
   <213> Artificial
<400) 7
   agccagtcct ggtgcagtac ggtgaggacg cttacaaccc ggtacgtgct gttgaattc 59
<210> 8
   <211> 59
   <212> DNA
   <213> Artificial
<400> 8
   ctgeaccagg actggctgaa tggcaaggaa tacaaatgca agagtacttc tagaatgta 59
<210> 9
   C211> 59
   <212> DNA
   <213> Artificial
<400> 9
   cgaaccagca gcgcattctg gaagtcgacg ttaccgcgcg ggtacattct agaagtact 59
<210> 10
   <211> 59
   <212> DNA
   <213> Artificial
<100> 10
   aatgcgctgc tggttcgtta caccaagaaa gtacccccaag tgtcaactcc aatcctgt 59
<210> 11
   <211> 59
   <212> DNA
   <213> Artificial
<400> 11
   gcggtaccgt taccgegcgg gtacatcata tgtgagacct ctacaggagt tggagttga 59
<210> 12
   <211> 59
   <212> DNA
   <213> Artificial
<400> 12
   cgcggtaacg gtaccgcgct ggaagttgac gaaacctacg ttccgaaaga atttaacgc 59
<210> 13
   <211> 64
   <212> DNA
   <213> Artificial
<400> 13
   tcgctagccc catccgcggg atgtcagcgt ggaaggtgaa ggtttccgcg ttaaattctt 60
   tcgg 64
<210> 14
   <211> 59
   <212> DNA
   <213> Artificial
<400> 14
   atcgagctca tgtaccgcg cggtaacgct agcgaacaaa aactcatctc agaagagga 59
<210> 15
   <211> 59
   <212> DNA
   <213> Artificial
<100> 15
   tattgctcgt gatggtgatg atgatgtgcg gccccattca gatcctcttc tgagatgag 59
<210> 16
   <211> 30
   <212> DNA
   <213> Artificial
<400> 16 30
   ctcgacggat ccttattgct cgtgatggtg 30
<210> 17
   <211> 22
   <212> DNA
   <213> Artificial
<400> 17 22
   taatacgact cactataggg ga 22
<210> 18
   <211> 19
   <212> DNA
   <213> Artificial
<400> 18 19
   gctagttatt gctcagcgg 19
<210> 19
   <211> 24
   <212> DNA
   <213> Artificial
<400> 19 24
   tcacatat gc aggtacagct acag 24
<210> 20
   <211> 25
   <212> DNA
   <213> Artificial
<400> 20
   ttcgctagcg gaagatacgg tacca 25
<210> 21
   <211> 25
   <212> DNA
   <213> Artificial
<400> 21
   aagagtactg aggtgaagct ggtgg 25
<210> 22
   <211> 27
   <212> DNA
   <213> Artificial
<400> 22
   gaagtcgaca gcgcgcttca gttccag 27
<210> 23
   <211> 15
   <212> PRT
   <213> Artificial
<400> 23
   Gly Gly Gly Gly Ser Gly Gly Gly Gly Ser Gly Gly Gly Gly Ser
   1 5 10 15
<210> 24
   <211> 20
   <212> DNA
   <213> Artificial
<400> 24
   ttcctcgagc aggttcagct 20
<210> 25
   <211> 58
   <212> DNA
   <213> Artificial
<400> 25
   tcgcgcccgg tttcatcagt tccgcaccgc tctgctgcag ctgaacctgc tcgatggaa 58
<210> 26
   <211> 58
   <212> DNA
   <213> Artificial
<400> 26
   actgatgaaa ccgggcgcga gcgtgaaaat cagctgcaaa gcgaccggct ataccttc 58
<210> 27
   <211> 44
   <212> DNA
   <213> Artificial
<400> 27
   cacccattcg atccaataat cgctgaaggt atagccggtc gctt 44
<210> 28
   <211> 59
   <212> DNA
   <213> Artificial
<400> 28
   attattggat cgaatgggtg aaacagcgtc cgggtcacgg cctggaatgg atcggtgaa 59
<210> 29
   <211> 58
   <212> DNA
   <213> Artificial
<400> 29
   acgttcgttg tagtcggtac ggccgctgcc cggcaggatt tcaccgatcc attccagg 58
<210> 30
   <211> 60
   <212> DNA
   <213> Artificial
<400> 30
   cgtaccgact acaacgaacg tttcaaaggc aaagcgacct tcaccggcga cgtttctagc 60
<210> 31
   <211> 60
   <212> D\A
   <213> Artificial
<400> 31
   ttcgctggte aggctagaca gtttcatata cgcggtgttg ctagaaacgt cgccggtgaa 60
<210> 32
   <211> 59
   <212> DNA
   <213> Artificial
<400> 32
   tgtctagcct gaccagcgaa gatagcgcgg tgtattactg cgcgaccggc accaccccg 39
<210> 33
   <211> 60
   <212> DNA
   <213> Artificial
<400> 33
   gctcacggtc accagggtgc cctgacccca gtaaccgaac ggggtggtgc cggtcgcgca 60
<210> 34
   <211> 59
   <212> DNA
   <213> Artificial
<400> 34
   gcaccctggt gaccgtgagc gcgactagta ccccgagcca taacagccat caggtgccg 59
<210> 35
   <211> 59
   <212> DNA
   <213> Artificial
<400> 35
   gtctctagag ccgctgttcg cggtcgggcc gcccgcgctc ggcacctgat ggctgttat 59
<210> 36
   <211> 58
   <212> DNA
   <213> Artificial
<400> 36
   cgaacagcgg ctctagagac atcgtgctga cccagagccc ggcgagcctg gcggtgtc 58
<210> 37
   <211> 60
   <212> DNA
   <213> Artificial
<400> 37
   ctgggagca cggcaggaga tggtcgcacg ctgacccaga gacaccgcca ggctcgccgg 60
<210> 38
   <211> 59
   <212> D\A
   <213> Artificial
<400> 38
   tctcctgccg tgcttcccag tccgtttcca cctcctccta cacctaeutg cactggtat 59
<210> 39
   <211> 56
   <212> DNA
   <213> Artificial
<400> 39
   gatcagcagt ttcggcggct gacccggttt ctgctgatac cagtgcatgt aggtgt 56
<210> 40
   <211> 59
   <212> DNA
   <213> Artificial
<400> 40
   asccgccgaa actgctgatc aaatatgcga gcaacctgga atctggtgtg ccggcgcgt 59
<210> 41
   <211> 59
   <212> DNA
   <213> Artificial
<400> 41
   gttcagggtg aagtcggtgc cgctgccaga accgctgaaa cgcgccggca caccagatt 59
<210> 42
   <211> 59
   <212> DNA
   <213> Artificial
<400> 42
   gcaccgactt caccctgaac atccacccgg tggaagaaga agataccgcg tattactat 59
<210> 43
   <211> 59
   <212> DNA
   <215> Artificial
<400> 43
   gccaccgaag gtacgcggga tttcccaaga gtgctggcaa tagtaatacg cggtatctt 59
<210> 44
   <211> 50
   <212> DNA
   <213> Artificial
<400> 44
   tcccgcgtac cttcggtggc ggcaccaaac tggaaatcaa agaattcgcc 50
<210> 45
   <211> 21
   <212> DNA
   <213> Artificial
<400> 45
   ggcgaattct ttgatttcca g 21
<210> 46
   <211> 21
   <212> DNA
   <213> Artificial
<400> 46
   ggcgaattct ttgatttcca g 21
<210> 47
   <211> 20
   <212> DNA
   <213> Artificial
<400> 47 20
   agccgccgaa actgctgatc 20
<210> 48
   <211> 20
   <212> DNA
   <213> Artificial
<400> 48
   gatcagcagt ttcggcggct 20
<210> 49
   <211> 20
   <212> DNA
   <213> Artificial
<400> 49 20
   cgaacagcgg ctctagagac 20
<210> 50
   <211> 20
   <212> DNA
   <213> Artificial
<400> 50 20
   gtctctagag ccgctgttcg 20
<210> 51
   <211> 20
   <212> DNA
   <213> Artificial
<400> 51
   gtaccgacta caacgaacgt 20
<210> 52
   <211> 20
   <212> DNA
   <213> Artificial
<400> 52
   acgttcgttg tagtcggtac 20

## Description

### FIELD OF THE INVENTION

This invention refers to the field of a recombinant genetically engineered antibody, more concretely, a linear single chain recombinant anti-CEA/CD3/CD28 trispecific antibody (scTsAb). This invention also refers DNA sequence coding for the said scTsAbs, plasmids containing the said sequences, and host cells containing the said plasmids.

### BACKGROUND OF THE INVENTION

The activation of T lymphocytes requires two kinds of signals *in vivo:* 1) the interaction between the MHC/antigen peptide complex on APC (antigen presenting cells) and the TCR/CD3 complex on T lymphocytes provides the first signal; and 2) the interaction between the co-stimulatory receptor on APC and the co-stimulatory molecule on T lymphocytes provides the second signal, a co-stimulatory signal. It was generally accepted that T lymphocytes cannot be activated fully in the presence of the first signal alone (Baxter and Hodgkin 2002; Bernard, Lamy et al. 2002).

There are two kinds of T lymphocytes including cytotoxic T lymphocytes (CTL) and T helper cells (TH). CTL is the major effector cell for a cellular immunological response, while TH participates in a cellular immunological response indirectly by secreting cytokines (such as interleukin-2 (IL-2)). As tumor immunity majors in cellular immunity, designing anti-tumor drugs to activate CTL specifically is of great importance in tumor immunotherapy (Foss 2002).

In practice, some recombinant anti-TAA/CD3 bispecific antibodies (BsAbs) have been designed to provide the first signal for CTL activation and retarget activated CTLs around tumor cells for killing them specifically. Among them, several ones have entered into clinic (Daniel, Kroidl et al. 1998; Holliger, Manzke et al. 1999; Loffler, Kufer et al. 2000; Manzke, Tesch et al. 2001; Manzke, Tesch et al. 2001; Dreier, Lorenczewski et al. 2002; Fang M 2002; Dreier, Baeuerle et al. 2003; Loffler, Gruen et al. 2003; Fang, Zhao et al. 2004). They have been proven to activate T lymphocytes specifically and induce obvious tumor specific cytolysis. However, most of them could not activate T lymphocytes fully and sometimes may result in activation of induced cell death (AICD) of T lymphocytes (Daniel, Kroidl et al. 1998).

To overcome the above defects, another kind of BsAb, anti-TAA/CD28 BsAb, was designed. Accompanied with anti-TAA/CD3 BsAb, CTLs are provided with dual activating signals, which induces more efficient tumor specific cytolysis. However, there are several disadvantages in the combinatorial use of two BsAbs. These include the duplicate steps in expression and purification, the consequential increase of production cost, and the partnership of two BsAbs in clinical medication. A trispecific antibody (TsAb) with three binding specificities (TAA, CD3, and CD28) might replace the above two BsAbs in providing dual activating signals (Jung, Brandl et al. 2001; Kodama, Suzuki et al. 2002) and be superior to them in expression, purification, and clinical medication.

Heretofore, there are three types of TsAb. These include 1) the chemically conjugated TsAb (Jung, Freimann et al. 1991; Tutt, Stevenson et al. 1991; French 1998; Wong, Vakis et al. 2000), 2) the recombinant polymeric TsAb (Atwell, Breheney et al. 1999; Dolezal, Pearce et al. 2000; Schoonjans, Willems et al. 2000; Schoonjans, Willems et al. 2000; Kortt, Dolezal et al. 2001; Schoonjans, Willems et al. 2001; Willems, Leoen et al. 2003), and 3) the single chain recombinant TsAb (scTsAb) (Li-ping, Ju-long et al. 2003). The third type of TsAb is believed to be superior to the others for its simplification in construction, expression, and purification. As carcinoembryonic antigen (CEA) is a broad-spectrum TAA (Ganjei, Nadji et al. 1988; Horie, Miura et al. 1996; Kuo, Tsai et al. 1996; Feil, Wechsel et al. 1999; Kammerer, Thanner et al. 2003; Shang-zhi, Chang-cheng et al. 2004), the scTsAb containing an anti-CEA antibody may be used in preventing or curing diverse tumors in clinic. Document EP 1 378 520 discloses a cyclic strand trispecific TAA/CD3/CD28 antibody.

### SUMMARY OF THE INVENTION

The introduction of anti-CEA antibody in an anti-CEA/CD3/CD28 scTsAb in this invention provides a convenience to distinguish tumor cells from normal cells *in vivo,* and avoid or decrease the non-specific killing by the activated T lymphocytes.

Another aspect of this invention is CEA is widely expressed on many tumor cells, and it also provides a broad application for curing or preventing different tumors in the future.

Even another aspect of this invention is it provides a method for constructing the scTsAb.

The amino acid sequence (SEQ ID NO: 1) of an murine anti-CEA single chain fragment of a variable region contained in the CEA-scTsAb is listed:

The amino acid sequence (SEQ ID NO: 2) of the anti-CD3 single chain fragment of the variable region contained in CEA-scTsAb is listed below:

The nucleic acid sequence (SEQ ID NO: 3) of CEA-scTsAb is listed as follows:

The amino acid sequence (SEQ ID NO: 4) of CEA-scTsAb is listed below:

Another aspect of this invention is that it provides a vector for expressing the CEA-scTsAb: CEA-scTsAb/pTRI.

However, in the context of this invention, other aspects and advantages of this invention are obvious to the ordinary persons engaged in the similar field, especially based on that disclosed in the " Example" part.

### BRIEF DESCRIPTION OF THE DRAWINGS

It will be understood that the drawings are intended to provide assistance to the present invention, which do not constitute a departure from the spirit and scope of the invention.

Fig.1. The diagram process of constructing a multi-cloning DNA frame with an overlapping PCR. The numbers from 2 to 11 represent different synthetic fragments of polymeric nucleic acids. The signs of " A, B, C, D, E, I , II, III, IV, UP, DOWN" represent the semi-finished products of constructions. The sign of "WHOLE" represents the final product.

Fig.2. Detection of the over-lapping PCR products by Agarose Gel electrophoresis. Lane 1: the product of the over-lapping PCR; Lane 2: DL2000 DNA marker (Dalian Takara Biotech.).

Fig.3. The sequence of the multi-cloning DNA frame

Fig.4. The process for constructing CAA-scTsAb

Fig.5. The diagram maps of vectors for constructing and expressing CEA-scTsAb.

Fig.6. Identification of the constructing process by Agarose Gel electrophoresis. Lane 1: PCR product amplified from the empty vector pTRI; Lane 2: PCR product amplified from vector CD28 V_{H}/pTRI; Lane 3: PCR product amplified from the vector CD3scFv/CD28 V_{H}/pTRI; Lane 4: PCR product amplified from the vector CEA-scTsAb/pTRI; Lane 5: DL2000 DNA marker (Dalian Takara Biotech.).

Fig.7. The diagram process for constructing murine anti-CEA scFv with the overlapping PCR is shown.

The numbers from 1 to 22 represent different synthetic fragments of polymeric nucleic acids. The signs of " A, B, C, D, E, F, G, H, I, J, K, a, b, c, d, e, f, g, I , II , III, IV, UP, DOWN" represent the semi-finished products of the construction. The sign of "WHOLE" represents the finished product.

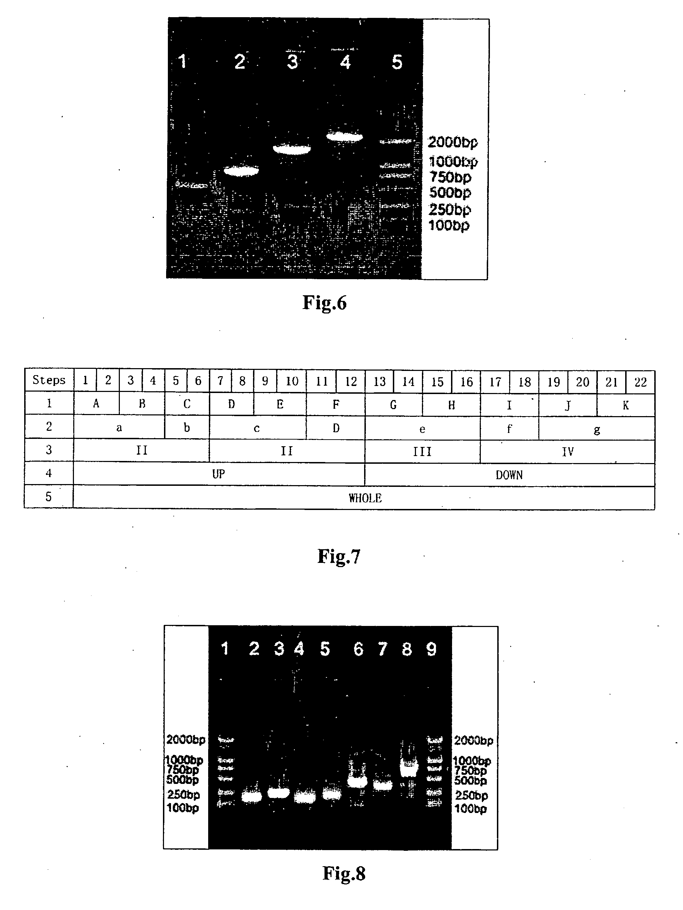

Fig.8. Detection of the over-lapping PCR product by Agarose Gel electrophoresis. Lane 1 and 9: DL2000 DNA markers (Dalian Takara Biotech.). Lane 2-5: semi-finished products: I, II , III, IV; Lane 6 and lane 7: semi-finished products UP and DOWN; Lane 8: the finished product WHOLE.

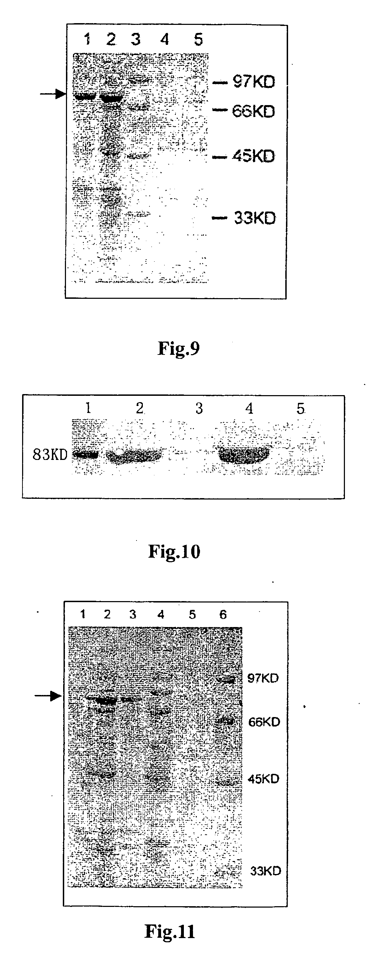

Fig.9. SDS-PAGE of the soluble expression of the CEA-scTsAb. Lane 1: the ultrasonic deposition of the CEA-scTsAb/pTRI expression; Lane 2: the ultrasonic supernatant of the CEA-scTsAb/pTRI expression; Lane 3: the protein molecular weight standards (Shanghai Biochemistry Institute); Lane 4: the ultrasonic deposition of the empty vector pTRI expression; Lane 5: the ultrasonic supernatant of the empty vector pTRI expression. The bands of the CEA-scTsAb are arrowed in corresponding lanes.

Fig.10. Western blotting of the soluble expression of CEA-scTsAb. Lane 1: protein molecular weight standards (NEB); Lane 2: the ultrasonic deposition of the CEA-scTsAb/ pTRI expression; Lane 3: the ultrasonic deposition of empty vector pTRI expression; Lane 4: the ultrasonic supernatant of the CEA-scTsAb/pTRI expression; Lane 5: the ultrasonic supernatant of the empty vector pTRI expression.

Fig.11. SDS-PAGE of CEA-scTsAb purified with DEAE anion exchange chromatography. Lane 1: the ultrasonic supernatant of the empty vector pTRI expression; Lane 2: the ultrasonic supernatant of the CEA-scTsAb/ pTRI expression; Lane 3: the flow-through of DEAE anion exchange chromatography; Lane 4: the NaCl elution of DEAE anion exchange chromatography; Lane 5: the NaOH elution of DEAE anion exchange chromatography; Lane 6: the protein molecular weight standards (Shanghai Biochemistry Institute). The bands of CEA-scTsAb are arrowed in corresponding lanes.

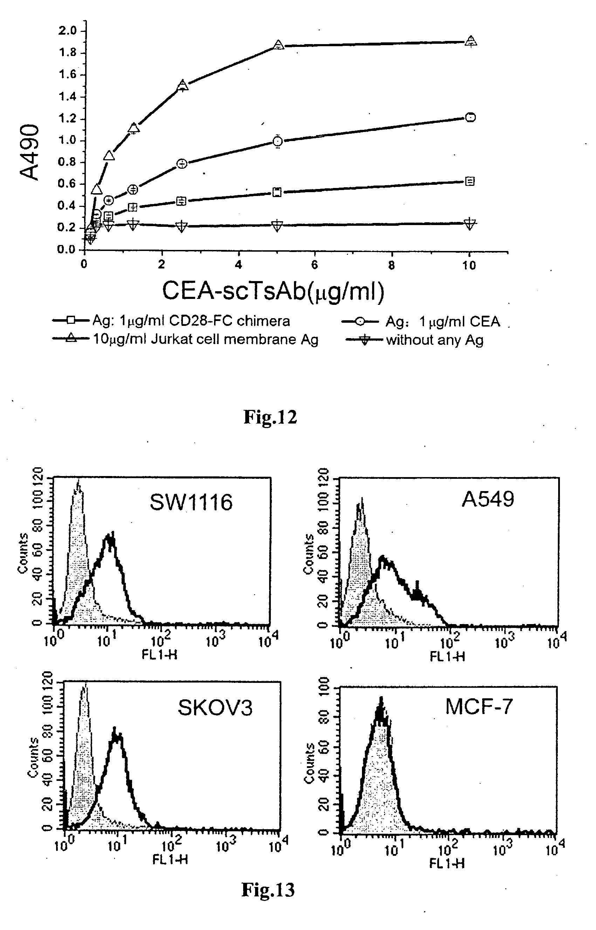

Fig.12. ELISA (enzyme linked immunosorbent assay) results of CEA-scTsAb. From top to bottom, the first curve: 10µg/ml Jurkat membrane antigen; the second one: 1µg/ml purified CEA (R&D); the third one: 1µg/ml CD28-Fc chimera (R&D); the fourth one with no antigen coated.

Fig.13. FCM of the binding of CEA-scTsAb to different tumor cells. The shadowed peak is the negative control with no CEA-scTsAb added.

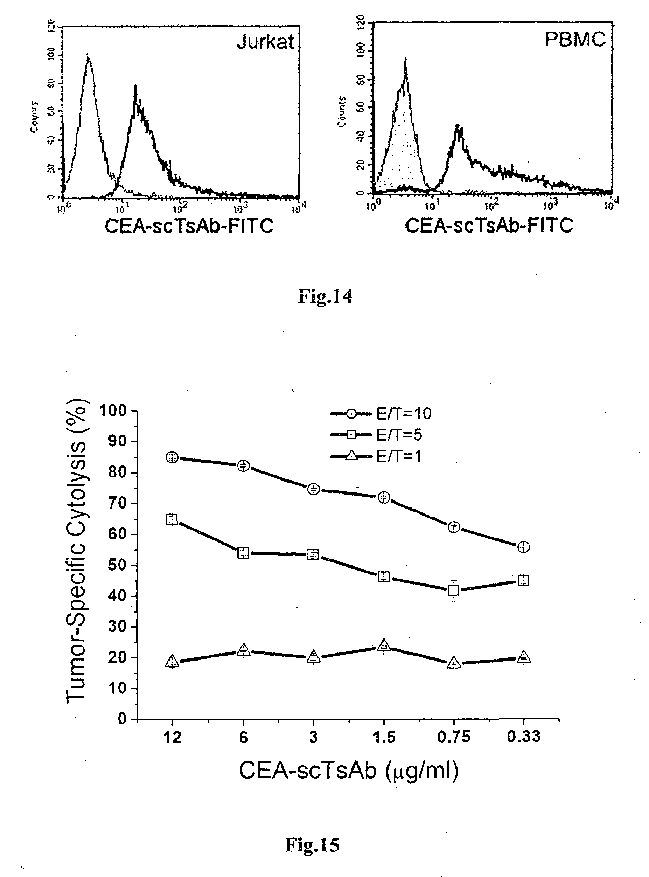

Fig.14. FCM of the binding of CEA-scTsAb to Jurkat cells and peripheral blood mononuclear cells (PBMC). The shadowed peak is the negative control with no CEA-scTsAb.

Fig.15. Effect of E/T ratio (effector cells/target cells) on tumor specific cytolysis induced by CEA-scTsAb as determined by the MTT assay. From top to bottom, the first curve: E/T=10; the second one: E/T=5; the third one: E/T=1. Effector cells: PBMC. Target cells: SW1116 tumor cells.

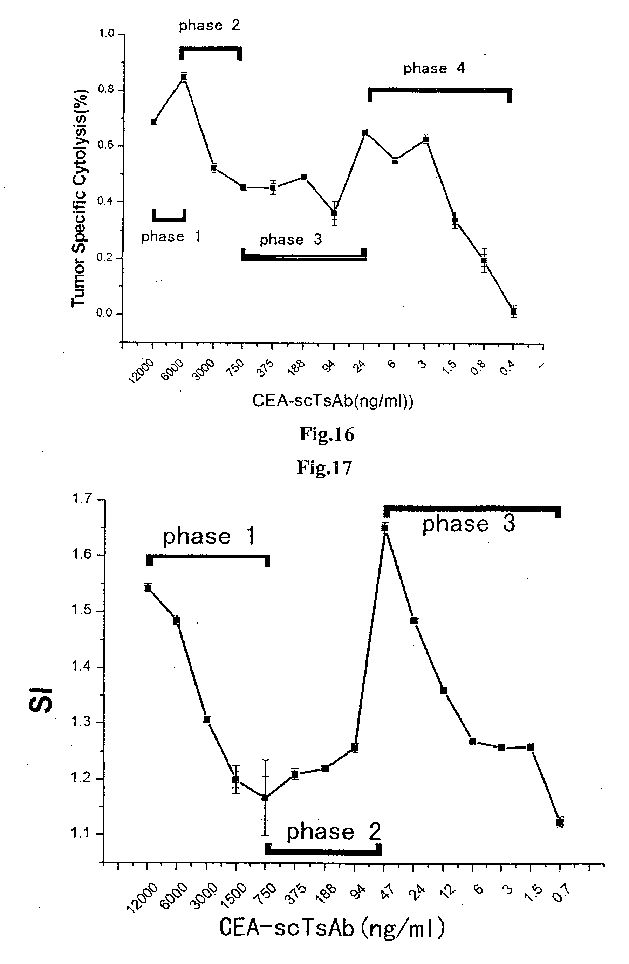

Fig.16. Effect of the CEA-scTsAb concentration on tumor specific cytolysis in a MTT assay.
There are four stepwise-phases for tumor specific cytolysis. In the first phase, from 6µg/ml to 12µg/ml, efficiency of the tumor specific cytolysis displays a negative correlation with the concentration of CEA-scTsAb and reached a peak at 6 µg/ml. In the second phase, from 750 ng/ml to 6µg/ml, it displayed a direct correlation and reaches the bottom at 750 ng/ml. In the third phase from 24 ng/ml to 750 ng/ml, it turns back into a negative correlation. In the fourth phase, from 24ng/ml to zero, the direct correlation appeared again.

Fig.17. Effect of the CEA-scTsAb concentration on the proliferation of effector cells as determined by MTT assay. There are three stepwise phases for stimulating index (SI). In the first phase from 750ng/ml to 12µg/ml, SI displays direct correlation with the concentration of CEA-scTsAb and reaches the bottom at 750ng/ml. In the second phase from 50ng/ml to 750ng/ml, it displayed a negative correlation and reached a peak at 50ng/ml. In the third phase from 50ng/ml to zero, it turns back into a direct correlation.

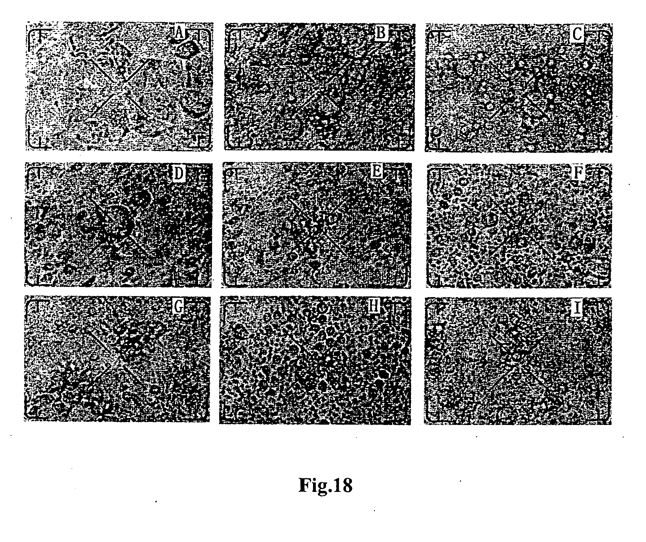

Fig.18. The morphological changes of mixed cells in the process of the tumor specific cytolysis induced by CEA-scTsAb. (A) SW1116 tumor cells after 20 hours of culturing. (B)-(I) the mixture of SW1116 (target cells) and PBMC (effector cells) added with CEA-scTsAb. E/T=5. (B) The adherent target cells begin to detach. (C) The effector cells aggregate on the surface of the target cells. (D) TsAbs appeared on the surface of the target cells. (E) Partial membrane of target cells breaks up. (F) The whole membranes of target cells break up. (G)-(I) The target cells break into fragments.

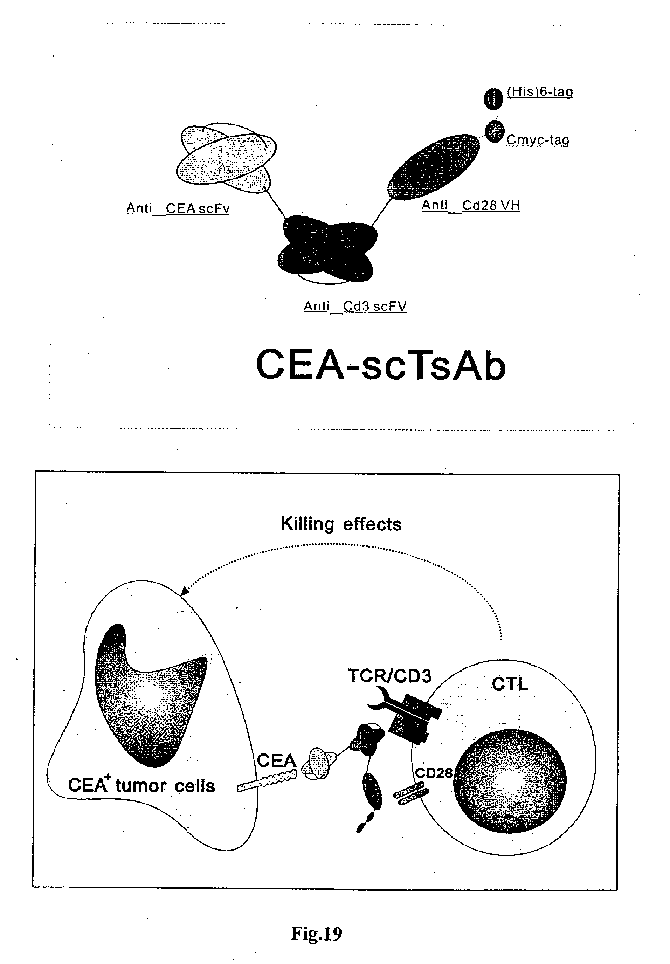

Fig.19. The mechanism diagram of the tumor specific cytolysis induced by CEA-scTsAb. The upper map: the structure of CEA-scTsAb; the lower map: The mechanism diagram of the tumor specific cytolysis induced by CEA-scTsAb; CEA-scTsAb binds to both target cell and the effector cell simultaneously, thus resulting in activation of the effector cell which is providing dual signals, which causes specific target cell killing.

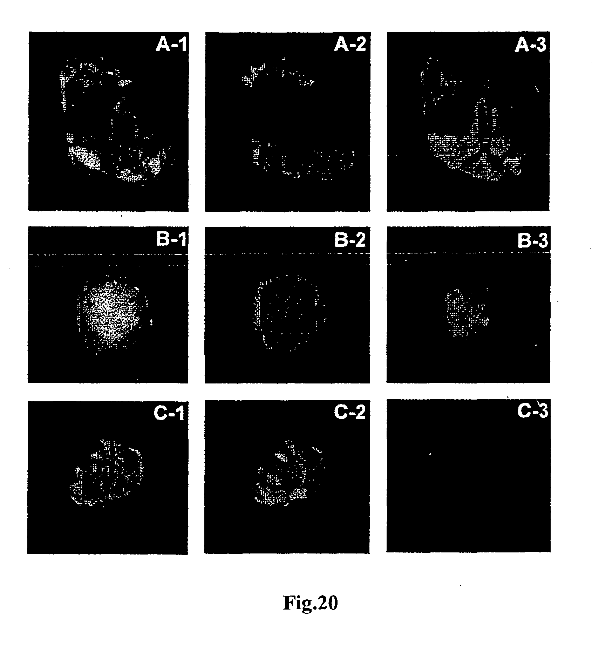

Fig.20. Micrographic photos of killed target cells (SW1116). Lines A, B, and C represent three different states of tumor cell death respectively: necrosis, late apoptosis and early apoptosis.

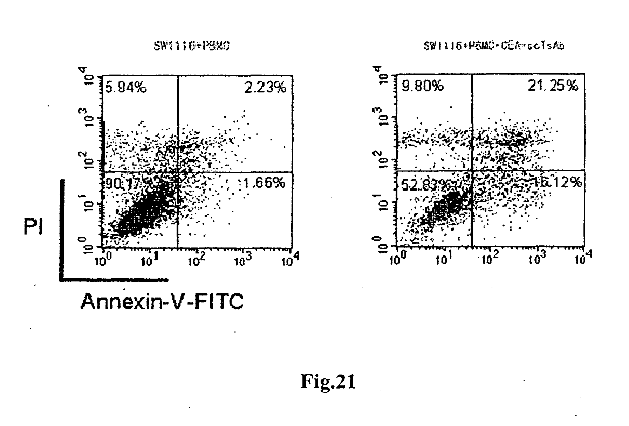

Fig.21. FCM (PI/Annexin V-FITC (Fluorescein Isothiocyanate) of tumor specific cytolysis. Four quadrants represent different states of tumor cells: live cells in the lower left quadrant (LL); early apoptosis cells in the lower right quadrant (LR); late apoptosis cells in the upper right quadrant (UR); necrosis cells in the upper left quadrant (UL). The sample with no CEA-scTsAb added: LL (90.17%), LR (1.66%), UL (5.94%), UR (2.23%); The sample added with 50ng/ml CEA-scTsAb: LL (52.83%), LR (16.12%), UL(9.8%), UR (21.25%).

### DESCRIPTION OF THE PREFERRED EMBODIMENT

In this invention, all terms are easy to understand for ordinary workers engaged in this field except which is explained explicitly. Here, some terms are described as below:
Linear single chain recombinant trispecific antibody is the linear single molecule constructed with three different antigen-binding specificities by a genetic engineering method. Specifically, a linear single chain recombinant anti-CEA/CD3/CD28 trispecific antibody is the single linear molecule constructed by fusing three different antibody fragments (anti-CEA antibody, anti-CD3 antibody, anti-CD28 antibody), which are spaced with two linkers (Fc linker and HSA linker) (Min Fang 2003). As a choice, c-myc tag and (His)6-tag can be added at the C terminal of the molecule for activity detection or further purification (Hengen 1995; Fan, Villegas et al. 1998). The antibody fragments mentioned here could be a single chain fragment of the variable region (scFv), an Fab fragment of an antibody, or a single domain antibody (V_{H} or V_{L}). More concretely, CEA-scTsAb is constructed by fusing an anti-CEA scFv, an Fc linker, an anti-CD3 scFv, an HSA linker, and an anti-CD28 V_{H} in tandem, a with c-myc tag and a His-6 tag at its C terminal. There are two advantages in this module:
   1. Based on a dual-signal activating model for T cells, it is endowed with the ability of activating T cells fully.
   2. As CEA is a broad-spectrum TAA, it is endowed with broad applications for curing or preventing many tumors in clinic.

The method for inducing the cytoplasmic soluble expression of CEA-scTsAb at low temperature mentioned in this invention requires that the host bacteria is induced with 0.4mM IPTG at 30°C. With this method, the ratio of inclusion bodies can be decreased remarkably and about 50% of expressed CEA-scTsAb is soluble. The soluble expression of CEA-scTsAb can be used in a further step of purification directly, without de-naturation or re-naturation, which can reduce the cost of production and improve the output.

The method of the single-step purification collecting flow-through of DEAE anion exchange chromatography requires that the soluble expression products are loaded into the column with the DEAE anion exchange resin at pH 8.0. Subsequently, almost all proteins of non-interest can be absorbed while most of the CEA-scTsAb flows through with an approximate 75% purity.

The operating procedure of this invention is listed below:
At first, the parent vector pTRI is constructed by introducing new special multi-cloning sites (MCS). Then the DNA fragment coding anti-CD28 V_{H} is amplified with PCR from the vector CD28 V_{H}/pTMF, at both ends of which a special pair of restriction sites, Nde *I*/Kpn I, is added. With the same method, the DNA fragment coding anti-CD3 scFv with restriction sites Scal/Sall, is prepared. The DNA fragment coding anti-CEA scFv with restriction sites Xhol/EcoRI, is cut from CEA scFv/pTMF. At last, all three fragments are introduced into pTRI in tandem to produce the final vector CEA scTsAb/pTRI. The DNA fragments coding anti-CEA scFv, anti-CD3 scFv and anti-CD28 V_{H} are arrayed in tandem from N terminus to C terminus.

With transformation into *E.coli* BL21(DE3) and induction with IPTG at low temperature (30°C), CEA-scTsAb is expressed solubly in cytoplasm. With the single step of DEAE anion exchange chromatography, it is primarily purified. The binding specificities to the three antigens (CEA, CD3, CD28) are detected by ELISA. The binding specificities to tumor cells are detected by FCM after conjugating CEA-scTsAb with FITC. The cytolysis of tumor cells and the proliferation of T lymphocytes induced by CEA-scTsAb are both analyzed by the MTT assay. The morphological changes of tumor cells are recorded by microphotography with an inverted microscope. The necrosis and apoptosis of tumor cells induced by CEA-scTsAb are visualized with a dual-color FCM, PI/annexin-V-FITC, and fluorescence-microscope.

### Examples:

### Example 1. Preparation of the DNA fragment containing multiple cloning sites by overlapping PCR

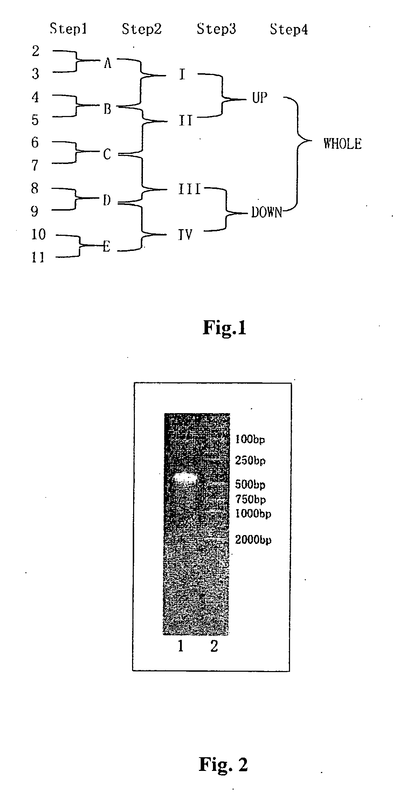

The schematic process is shown in Fig.1. All synthetic fragments used here are listed below:
1. 5'-TAT ACC ATG GGT CTC GAG-3' (SEQ ID NO:5)
2. 5'-TAT ACC ATG GGT CTC GAG ATG TAC CCG CGC GGT AAC ACT AGT GAA TTC AAC AGC ACG TA-3' (SEQ ID NO:6)
3. 5'-AGC CAG TCC TGG TGC AGT ACG GTG AGG ACG CTT ACA ACC CGG TAC GTG CTG TTG AAT TC-3' (SEQ ID NO:7)
4. 5'-CTG CAC CAG GAC TGG CTG AAT GGC AAG GAA TAC AAA TGC AAG AGT ACT TCT AGA ATG TA-3' (SEQ ID NO:8)
5. 5'-CGA ACC AGC AGC GCA TTC TGG AAG TCG ACG TTA CCG CGC GGG TAC ATT CTA GAAGTACT-3' (SEQ ID NO:9)
6. 5'-AAT GCG CTG CTG GTT CGT TAC ACC AAG AAA GTA CCC CAA GTG TCA ACT CCA ACT CCT GT-3' (SEQ ID NO:10)
7. 5'-GCG GTA CCG TTA CCG CGC GGG TAC ATC ATA TGT GAG ACC TCT ACA GGA GTT GGA GTT GA-3' (SEQ ID NO:11)
8. 5'-CGC GGT AAC GGT ACC GCG CTG GAA GTT GAC GAA ACC TAC GTT CCG AAA GAA TTT AAC GC-3' (SEQ ID NO:12)
9. 5'-TCG CTA GCC CCA TCC GCG GGA TGT CAG CGT GGA AGG TGA AGG TTT CCG CGT TAAATT CTT TCG G-3' (SEQ ID NO:13)
10. 5'-ATC GAG CTC ATG TAC CCG CGC GGT AAC GCT AGC GAA CAA AAA CTC ATC TCA GAA GAG GA-3' (SEQ ID NO:14)
11. 5'-TA TTG CTC GTG ATG GTG ATG ATG ATG TGC GGC CCC ATT CAG ATC CTC TTC TGA GAT GAG-3' (SEQ ID NO:15)
12.5'-CTCGACGGATCCTTATTGCTCGTGATGGTG-3' (SEQ ID NO:16)

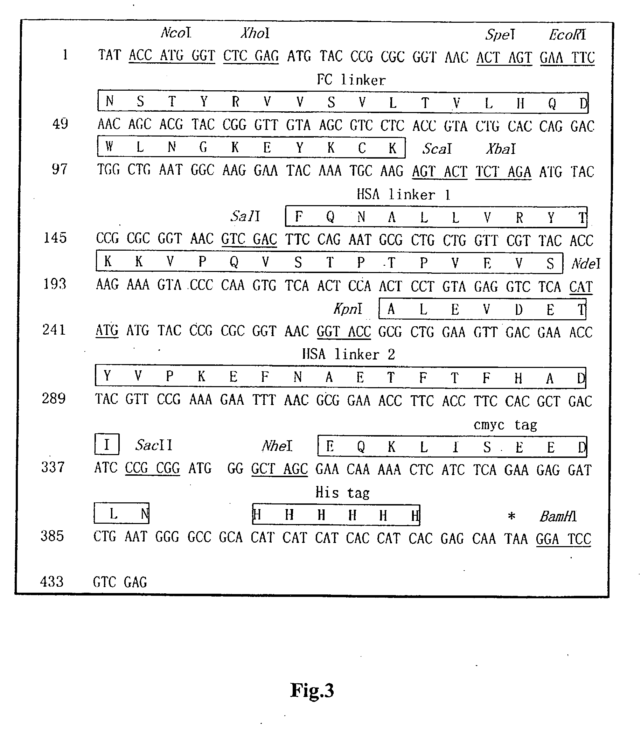

The operating steps:
**Step 1:** According to Fig.1, mix the fragments (from 2 to 11) in pairs and carry out the elongating reaction as below.
   Reaction mixture: the synthetic fragments, 1 µl (each); 10×PCR bluffer,2µl; dNTPs (2 mmol/ml each) (made in Dalian TaKaRa Biotechnology Co. Ltd.), 2µl; Taq (1U) (made in Dalian TaKaRa Biotechnology Co. Ltd.) 0.5µl; distilled water, 14µl.
   Reaction conditions: to pre-denature at 94°C for 1 minute; to denature at 94°C for 30 seconds; to anneal at 45°C for 30 seconds; to elongate at 72°C for 30 seconds; 10 cycles.
   All products are collected without any purification and applied in the next step directly.
**Step 2:** according to Fig.1, to mix the products (A, B, C, D, E) of step 1 in pairs and carry out the elongating reaction as below.
   Reaction mixture: the products of step 1, 10µl (each).
   Reaction condition: to pre-denature at 94°C for 1 minute; to denature at 94°C for 30 seconds; to anneal at 45°C for 30 seconds; to elongate at 72°C for 30 seconds; 10 cycles.
   All products are applied to agarose electrophoresis (1%) and purified by DNA Gel purifying Kit (Watson Biotech. Inc.).
**Step 3:** according to Fig.1, mix the products (I, II, III, IV) of step 2 in pairs and carry out the elongating reaction as below.
   Reaction mixture: the products of step 2, 1 µl (each); 10×PCR bluffer,2µl; dNTPs (2 mmol/ml each) (Dalian TaKaRa Biotechnology Co. Ltd.), 2µl; Taq (1U) (Dalian TaKaRa Biotechnology Co. Ltd.) 0.5µl; distilled water, 13µl.
   Reaction condition: to pre-denature at 94°C for 1 minute; to denature at 94°C for 30 seconds; to anneal at 45°C for 30 seconds; to elongate at 72°C for 30 seconds; 10 cycles.
   All products are applied to agarose electrophoresis (1%) and purified by DNA Gel Purifying Kit (Watson Biotech. Inc.).
**Step 4:** according to Fig.1, mix the products (UP, DOWN) of step 3 in pairs and carry out the amplifying reaction as below.
   Reaction mixture: the products of step 3, 1 µl (each); primers (synthetic fragments 1 and 12), 1µl; 10×PCR buffer, 2µl; dNTPs (2 mmol/ml each) (Dalian TaKaRa Biotechnology Co. Ltd.), 2µl; Taq (1 U) (Dalian TaKaRa Biotechnology Co. Ltd.) 0.5µl; distilled water, 12µl.
   Reaction condition: to pre-denature at 94°C for 1 minute; to denature at 94°C for 30 seconds; to anneal at 45°C for 30 seconds; to elongate at 72°C for 50 seconds; 25 cycles.
   The ultimate products (439bp) are applied to agarose electrophoresis (1%) (Fig.2.) and purified by the DNA Gel purifying Kit (Watson Biotech. Inc.). The sequence, restriction sites and its components are shown in Fig.3.

### Example 2. Construction of CEA-scTsAb

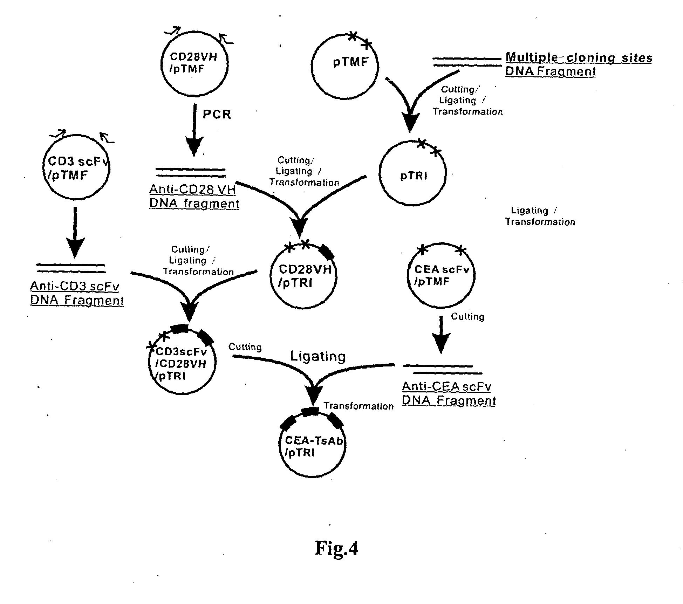

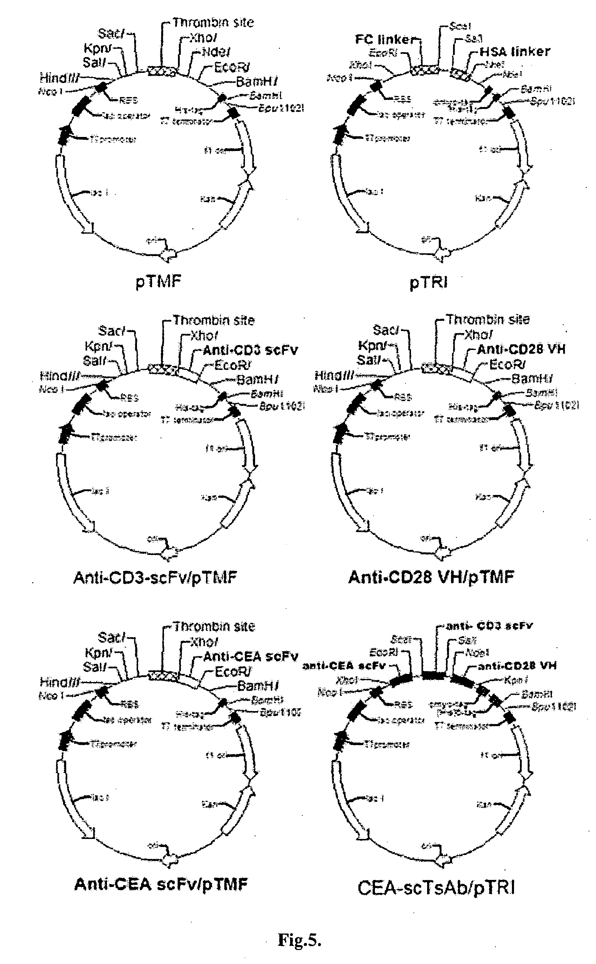

The process of construction is shown in Fig.4, and the schematic map of all vectors used in the process are listed in Fig. 5.

### (1) Construction of pTRI vector

The DNA fragment containing multiple cloning sites and empty vector pTMF (Zhang, Li et al. 2002) are both cut with NcoI/BamHI and ligated together. The products of ligation are transformed into *E.coli* strain TOP10 (Invitrogen). The plasmid isolated from the transformed bacterial cells is named pTRI.

Restriction enzyme digestion, ligation, preparation and transformation of TOP10 competent cells are carried out as below:
Restriction enzyme digesting reaction: in a volume of 20 µl, 1µg of pTMF or the DNA fragment containing multiple cloning sites are digested according to the operating manual from Promega company. The products are applied to agarose electrophoresis (1%) and purified by DNA Gel Purifying Kit (Watson Biotech. Inc.).
Ligating reaction: 50-100ng cut vector and 3-10 times(mol ratio) cut DNA fragments are mixed in a volume of 20 µl which contained 2µl 10×T4 DNA ligase buffer, 1U T4 DNA ligase (Dalian TaKaRa Biotechnology Co. Ltd.) and necessary distilled water. The ligating reaction is carried out at 16 °C overnight.
Preparation of TOP10 competent cells: inoculate the TOP10 bacteria (Invitrogen Co.) in 2ml LB medium ((10g/l tryptone (GIBCO Co.), 5g/l yeast extract (GIBCO Co.), 5g/l NaCl, pH 7.5)), and incubate overnight at 37°C with shaking. Transfer to 20-40 ml LB medium at a ratio of 1:100, incubate at 37°C with shaking to reach A600 of 0.3-0.4 (about 2.5 hour). Chill on ice for 15 minutes and centrifuge at 4°C at 4000 rpm for 10 minutes. The pellet is resuspended in 10 ml of pre-chilled 0.1 mol/l CaCₗ₂ (Sigma Co.) and chilled on ice for 20 minutes. After a second centrifugation at 4°C at 4000 rpm for 10 minutes, the pellet is gently resuspended in 1-2ml of pre-chilled 0.1 mol/l CaCₗ₂ solution with 12% glycerol. Divide into aliquots of 200 µl per EP tube, store at -80°C.
Transformation: the ligating mixture is added to 200 µl competent cells. After being mixed gently and chilled on ice for 30 minutes, it is put in a 42 degrees (Celsius) water bath for 100 seconds then chilled on ice for 2 minutes. After adding 0.8 ml LB medium into the mixture, shake it at 37°C (<150 rpm) for 45 minutes to recover the cells. The cells are centrifuged at 10,000 rpm for 1 minute, re-suspended in 50∼100µl LB medium, spread onto the LB-K plate (10g/l tryptone, 5g/l yeast extract, 5g/l NaCl, 15g/l agar (SIGMA Co.), 50 µg/ml kanamycin (SIGMA Co.), pH 7.5), and incubated at 37°C overnight.
Selection of positive clones: pick the single clones on the LB-K plate and transfer them into 2ml LB-K medium (10g/l tryptone, 5g/l yeast extract, 5g/l NaCl, 15g/l agar (SIGMA Co.), 50 µg/ml kanamycin (SIGMA Co.), pH 7.5) separately. After shaking at 37°C overnight, the plasmids are isolated with a Plasmid Isolating Kit.(Watson Biotechnologies, Inc) according to the standard manual. The positive clones are identified by PCR with the isolated plasmids as the templates. PCR reaction mixture: 0.1∼1 µ I plasmid DNA (about 20-200ng); 10 pmol upstream primer (T7-up: 5'-TAATACGACTCACTATAGGGGA-3') (SEQ ID NO:17); 10 pmol down stream primer (T7-down: 5'-GCTAGTTATTGCTCAGCGG-3') (SEQ ID NO:18); 2 µl 10×Taq buffer; 2 µl 2mmol/ml dNTPs; 1 U Taq; 12 µl distilled-water. The PCR reaction condition: pre-denature at 94°C for 5 minutes; denature at 94°C for 40 seconds; anneal at 53 °C for 40 seconds; elongate at 72°C for 40 seconds; 25 cycles. At last 5 µl PCR product are applied to agarose electrophoresis (1%). As shown in Fig.6, the PCR product is about 500bp.

### (2). Construction of CD28 V_{H}/pTRI,

The DNA fragment coding anti-CD28V_{H} is amplified from CD28 V_{H}/pTMF (Cheng, Wang et al. 2002) with P1 (P1: 5'-TCACATATGCA GGTACAGC TACAG-3') (SEQ ID NO: 19) as the up-stream primer and P2 (P2: 5'-TTCGCTAGCGGAAGATACGGTA CCA-3') (SEQ ID NO; 20) as the down-stream primer. The restriction sites NdeI/NheI are introduced at the 5'end and 3' end respectively during the process of PCR.

PCR reaction mixture: 1 µl primers(each); 2µl dNTP (2mmol/ml each); 2 µl 10×pfu buffer; 100ng CD28 V_{H}/pTRI, plasmid; 0.3µl Pfu (Promega Co.); add distilled water to the volume of 20µl. PCR reaction condition: to pre-denature at 94°C for 3 minutes; to denature at 94°C for 30 seconds; to anneal at 55°C for 30 seconds; to elongate at 72°C for 50 seconds; 25 cycles. The PCR products are purified by agarose electrophoresis (1%) and DNA Gel purification kit (Watson Biotech. Inc.).

The above PCR product and pTRI plasmid are cut with NdeI/Nhel (Promega Co.) at the same time. The cutting of the PCR product (about 350bp) and of pTRI (about 5300bp) are ligated together and transformed into TOP10 *E.coli* strain. The plasmids isolated from the positive clones are named as CD28 V_{H}/pTRI, which are identified by PCR with the product of about 750bp (As shown in Fig.6). All operating procedures needed here come from step (1).

### (3) Construction of CD3 scFv/ CD28 V_{H}/pTRI

The DNA fragment coding anti-CD3 scFv is amplified from CD3 scFv/pTMF (Liu XF 1996)with P1 (P1: 5'-AAGAGTACTGAGGTGAAGCTGGTGG-3') (SEQ ID NO: 21) as the up-stream primer and P2 (P2: 5'-GAAGTCGACAGCGCGCTTCAGTTCCAG-3') (SEQ ID NO: 22) as the down-stream primer. The restriction sites, Scal and Scall, are introduced at the 5'end and 3' end respectively during the process of PCR.

PCR reaction mixture: 1µl primers(each); 2µl dNTP (2mmol/ml each);2 µl 10×pfu buffer; 100ng CD28 V_{H}/pTRI, plasmid; 0.3µl Pfu (Promega Co.); add distilled water to the volume of 20µl. PCR reaction condition: to pre-denature at 94°C for 3 minutes; to denature at 94"C for 30 seconds; to anneal at 55°C for 30 seconds; to elongate at 72°C for 50 seconds; 25 cycles. The PCR products are purified by agarose electrophoresis (1%) and DNA Gel purification kit (Watson Biotech. Inc.).

The above PCR product and CD28 V_{H} /pTRI plasmid are cut with Scal/Scall (Promega Co.) at the same time. The cutting product of PCR (about 750bp) and that of CD28 V_{H} /pTRI (about 5700bp) are ligated together and transformed into TOP 10 *E.coli* strain. The plasmids isolated from the positive clones are named as CD3 scFv/CD28 V_{H}/pTRI, which are identified by PCR with the product of about 1400bp (As shown in Fig.6). All operating procedures needed here come from step (1).

### (4) Construction of CEA-scTsAb/pTRI

Construction of anti-CEA scFv by overlapping PCR:
Anti-CEA scFv is designed by Linking V_{H} (the variable region of heavy chain) and V_{L} (the variable region of light chain) of anti-CEA monoclonal antibody(Koga, Kanda et al. 1990) with a special polypeptide GGGGSGGGGSGGGGS) (SEQ ID NO: 23). The whole amino acid sequence of anti-CEA scFv is back translated into a DNA sequence according to the *E.coli* preferred codon table(Nakamura, Gojobori et al. 2000), which is split into 22 complemental oligo-nucleotides. The 22 oligo-nucleotides listed below are synthesized and assembled into the whole DNA fragment coding anti-CEA scFv by overlapping PCR.
   1. 5'-TTCCTCGAGCAGGTTCAGCT-3' (SEQ ID NO:24)
   2. 5'-TCGCGCCCGGTTTCATCAGTTCCGCACCGCTCTGCTGCAGCTGAACCTGCTCGAGGAA-3' (SEQ ID NO:25)
   3. 5'-ACTGATGAAACCGGGCGCGAGCGTGAAAATCAGCTGCAAAGCGACCGGCTATACCTTC-3' (SEQ ID NO:26)
   4. 5'-CACCCATTCGATCCAATAATCGCTGAAGGTATAGCCGGTCGCTT-3' (SEQ ID NO:27)
   5. 5'-ATTATTGGATCGAATGGGTGAAACAGCGTCCGGGTCACGGCCTGGAATGGATCGGTGAA-3 (SEQ ID NO:28)
   6. 5'-ACGTTCGTTGTAGTCGGTACGGCCGCTGCCCGGCAGGATTTCACCGATCCATTCCAGG-3' (SEQ ID NO:29)
   7. 5'-CGTACCGACTACAACGAACGTTTCAAAGGCAAAGCGACCTTCACCGGCGACGTTTCTAGC -3' (SEQ ID NO:30)
   8. 5'-TTCGCTGGTCAGGCTAGACAGTTTCATATACGCGGTGTTGCTAGAAACGTCGCCGGTGAA-3' (SEQ ID NO:31)
   9. 5'-TGTCTAGCCTGACCAGCGAAGATAGCGCGGTGTATTACTGCGCGACCGGCACCACCCCG-3' (SEQ ID NO:32)
   10. 5'-GCTCACGGTCACCAGGGTGCCCTGACCCCAGTAACCGAACGGGGTGGTGCCGGTCGC GCA-3' (SEQ ID NO:33)
   11. 5'-GCACCCTGGTGACCGTGAGCGCGACTAGTACCCCGAGCCATAACAGCCATCAGGTGCC G-3' (SEQ ID NO:34)
   12. 5'-GTCTCTAGAGCCGCTGTTCGCGGTCGGGCCGCCCGCGCTCGGCACCTGATGGCTGTTA T-3' (SEQ ID NO:35)
   13. 5'-CGAACAGCGGCTCTAGAGACATCGTGCTGACCCAGAGCCCGGCGAGCCTGGCGGTGT C-3' (SEQ ID NO:36)
   14. 5'-CTGGGAAGCACGGCAGGAGATGGTCGCACGCTGACCCAGAGACACCGCCAGGCTCGC CGG-3' (SEQ ID NO:37)
   15. 5'-TCTCCTGCCGTGCTTCCCAGTCCGTTTCCACCTCCTCCTACACCTACATGCACTGGTAT-3 (SEQ ID NO:38)
   16. 5'-GATCAGCAGTTTCGGCGGCTGACCCGGTTTCTGCTGATACCAGTGCATGTAGGTGT-3' (SEQ ID NO:39)
   17. 5'-AGCCGCCGAAACTGCTGATCAAATATGCGAGCAACCTGGAATCTGGTGTGCCGGCGCG T-3' (SEQ ID NO:40)
   18. 5'-GTTCAGGGTGAAGTCGGTGCCGCTGCCAGAACCGCTGAAACGCGCCGGCACACCAGA TT-3' (SEQ ID NO:41)
   19. 5'-GCACCGACTTCACCCTGAACATCCACCCGGTGGAAGAAGAAGATACCGCGTATTACTAT-3' (SEQ ID NO:42)
   20. 5'-GCCACCGAAGGTACGCGGGATTTCCCAAGAGTGCTGGCAATAGTAATACGCGGTATCTT-3' (SEQ ID NO:43)
   21. 5'-TCCCGCGTACCTTCGGTGGCGGCACCAAACTGGAAATCAAAGAATTCGCC-3' (SEQ ID NO:44)
   22. 5'-GGCGAATTCTTTGATTTCCAG-3' (SEQ ID NO:45)
   S1) 5'-GGCGAATTCTTTGATTTCCAG-3' (SEQ ID NO:46)
   S17) 5'-AGCCGCCGAAACTGCTGATC-3' (SEQ ID NO:47)
   S16) 5'-GATCAGCAGTTTCGGCGGCT-3' (SEQ ID NO:48)
   S13) 5'-CGAACAGCGGCTCTAGAGAC-3' (SEQ ID NO:49)
   S12) 5'-GTCTCTAGAGCCGCTGTTCG-3' (SEQ ID NO:50)
   S7) 5'-GTACCGACTACAACGAACGT-3' (SEQ ID NO:51)
   S6) 5'-ACGTTCGTTGTAGTCGGTAC-3' (SEQ ID NO:52)

The operating steps:
**Step1:** according to Fig.7, mix the fragments (from 1 to 22) in pairs and carry out the elongating reaction as below.
   Reaction mixture: the synthetic fragments, 1 µl (each); 10×PCR buffer, 2µl; dNTPs (2 mmol/ml each) (Dalian TaKaRa Biotechnology Co. Ltd.), 2µl; Taq (1U) (Dalian TaKaRa Biotechnology Co. Ltd.) 0.5µl; distilled water, 14µl.
   Reaction condition: to pre-denature at 94°C for 1 minute; to denature at 94°C for 30 seconds; to anneal at 45°C for 30 seconds; to elongate at 72°C for 30 seconds; 10 cycles.
   All products are collected without any purification and applied in next step directly.
**Step2:** according to Fig.7, mix the products (A, B, D, E, G, H, J, K) of step 1 in pairs and carry out the elongating reaction as below.
   Reaction mixture: the products of step 1, 10 µl (each).
   Reaction condition: to pre-denature at 94°C for 1 minute; to denature at 94°C for 30 seconds; to anneal at 45"C for 30 seconds; to elongate at 72°C for 30 seconds; 10 cycles.
   All products are applied to agarose electrophoresis (1%) and purified by DNA Gel purifying Kit (Watson Biotech. Inc.). The fragments of "a" and "g" are about 120bp; the fragments of "c" and "e" are about 170bp; the fragments of "b", "d" and "f' are about 100bp.
**Step3:** according to Fig.7, mix the products (a, b, c, d, e, f, g) of step 2 in pairs and carry out the amplifying reaction as below. Primer S1 and S6 correspond to the pair of "a" and "b"; Primer S7 and S12 correspond to the pair of "c" and "d"; Primer S13 and S16 correspond to "e", Primer S17 and 22 correspond to the pair of "f" and "g".
   Reaction mixture: the product of step 2, 1 µl(each); primers, 1µl (each); 10×PCR buffer,2µl; dNTPs (2 mmol/ml each) (Dalian TaKaRa Biotechnology Co. Ltd.), 2µl; Taq (1U) (Dalian TaKaRa Biotechnology Co. Ltd.) 0.5µl; distilled water, 12µl.
   Reaction condition: to pre-denature at 94°C for 1 minute; to denature at 94°C for 30 seconds; to anneal at 45°C for 30 seconds; to elongate at 72°C for 30 seconds; 25 cycles.
   All products are applied to agarose electrophoresis (1%) and purified by DNA Gel purifying Kit (Watson Biotech. Inc.). The fragment of I is about 200bp; the fragment of II is about 250bp; the fragment of III is about 140bp; the fragment of IV is about 230bp.
**Step4:** according to Fig.7, mix the products (I, II, III, IV) of step 3 in pairs and carry out the amplifying reaction as below. Primer S1 and S12 correspond to the pair of I and II; Primer S13 and 22 correspond to the pair of III and IV.
   Reaction mixture: the product of step 3, 1 µl (each); primers, 1µl (each); 10×PCR buffer, 2µl; dNTPs (2 mmol/ml each) (Dalian TaKaRa Biotechnology Co. Ltd.), 2µl; Taq (1U) (Dalian TaKaRa Biotechnology Co. Ltd.) 0.5µl; distilled water, 12µl.
   Reaction condition: to pre-denature at 94°C for 1 minute; to denature at 94°C for 30 seconds; to anneal at 45°C for 30 seconds; to elongate at 72°C for 30 seconds; 25 cycles.
   All products are applied to agarose electrophoresis (1%) and purified by DNA Gel purifying Kit (Watson Biotech. Inc.). The fragment of UP is about 430bp; the fragment of DOWN is about 340bp.
**Step5:** according to Fig.7, mix the products (UP and DOWN) of step 4 in pair and carry out the amplifying reaction as below. Primer S1 and 22 correspond to the pair of UP and DOWN.
   Reaction mixture: the product of step 4, 1 µl (each); primers, 1µl (each); 10×PCR buffer, 2µl; dNTPs (2 mmol/ml each) (made in Dalian TaKaRa Biotechnology Co. Ltd.), 2µl; Taq (1U) (made in Dalian TaKaRa Biotechnology Co. Ltd.) 0.5µl; distilled water, 12µl.
   Reaction condition: to pre-denature at 94°C for 1 minute; to denature at 94°C for 30 seconds; to anneal at 45°C for 30 seconds; to elongate at 72°C for 60 seconds; 25 cycles.
   All products are applied to agarose electrophoresis (1%) and purified by DNA Gel purifying Kit (Watson Biotech. Inc.). The fragment of WHOLE is about 750bp.
   The schematic process of above operations is shown in Fig. 7 and the results of identifying PCR is shown in Fig.8.
   The above PCR product and pTMF plasmid are cut with XhoI/EcoRI (Promega Co.) at the same time. The cutting product of PCR (about 750bp) and that of pTMF (about 5200bp) are ligated together and transformed into TOP10 *E.coli* strain. The plasmids isolated from the positive clones are named as CEA scFv/pTMF, which are identified by PCR with the product of about 750bp. All operating procedures needed here come from step (1).
   The CEA scFv/pTMF plasmid and the CD3 scFv/CD28 V_{H}/pTRI plasmid are cut with XhoI/EcoRI (Promega Co.) at the same time. The cutting product of the former (about 750bp) and that of the latter (about 6000bp) are ligated together and transformed into TOP10 *E.coli* strain. The plasmids isolated from the positive clones are named CEA scTsAb/pTRI, which are identified by PCR with the product of about 2100bp (As shown in Fig.6). All operating procedures needed here come from step (1).

### Example 3: Soluble cytoplasmic expression of CEA-scTsAb induced at lower temperature.

### (1) Transformation of CEA scTsAb/pTRI into BL21 (DE3)(Novagen) E.coli strain.

The competent BL21 (DE3) cells are prepared referring to the method in example 2. The plasmid CEA scTsAb/pTRI) is isolated with plasmid isolating kit (Watson Biotech.Inc.). The subsequent procedures of transformation and identification of positive clones are performed according to example 2.

### (2) Induced expression at lower temperature

The single clone of BL21 (DE3) containing CEA-scTsAb/pTRI is picked up from the LB-K plate and inoculated in 5ml LB-K medium. After being cultured at 37°C with shaking overnight, the culture is transferred into 250 ml LB-K medium at a ratio of 1/100, shake at 37°C to reach A600 of 0.6. IPTG (Takara Biotech. (Dalian)) is added to the final concentration of about 0.4mmol/l to induce soluble expression at 30°C for 4 hours. The bacterial cells are harvested by centrifuging at 12,000 rpm for 10 minutes and then re-suspended in phosphate buffered saline (PBS: 8g NaCl, 0.2gKCl, 1.44g Na₂HPO₄, 0.24g KH₂PO₄, pH7.4, 1 liter) (1/5 volume of culture medium). Thus, cytoplasmic soluble CEA-scTsAb is released into the supernatant produced by centrifuging ultrasonic-lyzed cells. Furthermore, soluble expression and inclusion body expression of CEA-scTsAb are detected by reducing SDS-PAGE and Western-blotting according to "molecular cloning: a laboratory manual"(Sambrook and Russell 2001). The results are photographed with Alpha-Image 2200 Documentation and analysis system (American Alpha Innotech Company). As shown in Fig.10, the soluble expression of CEA-scTsAb occupies about 70% of its total expression. As ultrasonic supernatant can be applied in further steps of purification and *in vitro* activity assays directly, with no need of denaturation or renaturation, the cost and time of production would be saved remarkably.

### Example 4: Purification of CEA-scTsAb by a single step of DEAE anion exchange chromatography.

250ml culture medium containing bacterial cells expressing CEA-scTsAb are centrifuged at 12,000rpm at 4°C for 10 minutes. The pellet is resuspended in 50ml equilibrium buffer (20mmol/l NaCl, 20mmol/l Tris-HCl, pH 8.0) of DEAE anion exchange chromatography for further sonication. After a second centrifugation at 12,000 rpm at 4°C for 10 minutes, the supernatant containing solubly expressed CEA-scTsAb is applied in the purifying step directly.

20ml of DEAE anion exchange resin (Amersham Bioscience) is suspended in 100ml equilibrium buffer and packed into a 16×20cm column (Shanghai Hua-mei). The column is equilibrated with 5 volumes of equilibrium buffer at a rate of 1 ml/minute. The above supernatant is then loaded at a rate of 0.25ml/minute. Purified CEA-scTsAb exists in the flow-through fraction. The column is washed or eluted with 2 volumes of eluting buffer (500mmol/l NaCl, 20mmol/l Tris-HCl, pH 8.0) at a rate of 0.25ml/minute and cleaned with 2 volumes of 500mmol/l NaOH at a rate of 0. 5ml/minute. During the regenerating step, the column is equilibrated with 2 volumes of equilibrium buffer at a rate of 1 ml/minute.

Reducing SDS-PAGE of the above flow through fraction, the result of purification is shown in Fig.11. As a result, most of the bacterial protein in the supernatant can be removed by a single step of DEAE anion exchange chromatography. CEA-scTsAb occupied 70% of the flow-through fraction.

The purified sample is then dialyzed against PBS at 4°C overnight. The protein concentration is quantified by Bradford method(Ausubel 1999). After supplementing sodium azide (0.05%(W/V)) and fucose (0.15mol/l), the dialyzed solution is divided into 1ml aliquots and stored at-80°C.

### Example 5: Detection of the binding specificity to three antigens (CEA, CD3, CD28) by ELISA.

**Preparation of Jurkat cell membrane antigen:** 5×10⁶ Jurkat cells (American type culture collection, ATCC, TIB-152) are harvested by centrifuging at 1000g for 10 minutes. The cell pellet is suspended in 0.5ml PBS and lysed by ultra-sonication. The supernatant of the ultrasonication produced by centrifuging at 12,000rpm for 10 minutes is supplemented with sodium azide (0.05%(W/V)) and fucose (0.15mol/l), divided into 100µl aliquots and stored at -80°C.

### ELISA:

(1) Coating: purified CEA (R&D), rhCD28-Fc chimera(R&D) and the above purified Jurkat membrane antigen are diluted in coating buffer (1.36g Na2CO3, 7.35g NaHCO3, 1 liter, pH 9.2) in the concentration of 1µg/ml (CEA and rhCD28-Fc chimera) or 10µg/ml (Jurkat membrane antigen) and coated with 100µl/well in 96 well ELISA plate (Nunc). The plate is placed at 37°C for 2 hours or at 4"C overnight.
(2) Blocking: the coating plate is washed with PBS 1-2 times and the blocking buffer (PBS-1% BSA (Bovine Serum Albumin, w/v) is added at 200µl/well. The plate is placed at 37°C for 2 hours.
(3) Addition of Samples: the blocked plate is washed with PBS 3 times and the diluted samples in PBS is added with 100µl/well. The samples of CEA-scTsAb are serially diluted from the primary concentration of 10µg/ml 6 times. The plate is placed at 37°C for 2 hours.
(4) Addition of the primary antibody: the sample buffer in the plate is washed with PBS-T (PBS-0.05%Twen-20 (w/v)) 3 times and 1/1000 diluted mouse anti-cmyc tag monoclonal antibody (Santa Crutz) is added at 100µl/well. The plate is placed at 37°C for 2 hours.
(5) Addition of the secondary antibody: the primary antibody in the plate is washed with PBS-T 3 times and 1/1000 diluted HRP (horse-radish peroxidase) conjugated goat anti-mouse IgG (Santa Crutz) is added at 100µl/well. The plate is placed at 37°C for 2 hours.
(6) Visualization: the secondary antibody in the plate is washed with PBS-T 5 times and the visualizing solution containing 10ml substrate buffer (36.6 g Citric Acid, monohydrate, 113.5g Potassium dibasic phosphate, 1 liter, pH6.0) and 4mg OPD (orthofenylenediamin.diHCl, Sigma) is added at 100µl/well. The plate is placed at room temperature in the dark for 2 0 minutes.
(7) Stop of reaction: 1mol/l HCl is added with 100µl/well to stop the reaction.
(8) Measurement: the absorbent result is read at 490nM.

As shown in Fig.12, CEA-scTsAb binds to three antigen, CEA, CD3 rich Jurkat membrane antigen, and rhCD28-Fc chimera very specifically.

### Example 6: Detection of the binding specificity to tumor cells by FCM

An indirect FCM method is used to detect the binding to various tumor cells. The sources of these tumor cells are listed below.

| Designation | Source | ATCC Number |
|---|---|---|
| A549 | Lung, carcinoma | CCL-185 |
| MCF-7 | Human, mammary gland, breast adenocarcinoma | HTB-22 |
| SK-OV-3 | Ovary, adenocarcinoma | HTB-77 |
| SW 1116 | Colorectal, adenocarcinoma | CCL-233 |

Operating:
(1) Culture and collection of tumor cells: three type of tumor cells (A549, MCF-7, SK-OV-3) are cultured in 10% fetal calf serum (FCS)-containing RPMI 1640 medium (Gibco) plus antibiotics (100 U penicillin) in a humidified 5 % CO2 incubator at 37°C. SW1116 is cultured in 10% fetal calf serum (FCS)-containing L15 medium (Gibco) plus antibiotics (100 U penicillin) in a humidified 5 % CO2 incubator at 37°C. 5×10⁵ tumor cells in the exponential phase are collected by centrifuging at 1000g for 10 minutes and suspended in 100µl PBS.
(2) Incubation of CEA-scTsAb with tumor cells: CEA-scTsAb is added into the above tumor cells containing-PBS solution at a final concentration of 10µg/ml. The isotype control is set for each tumor cell. The cell suspensions are incubated at 4°C for 30 minutes.
(3) Incubation of the primary antibody, mouse anti-cmyc tag monoclonal antibody (Santa Crutz), with tumor cells: unbound CEA-scTsAbs are removed by discarding the supernatant after centrifuging at 1000g for 10 minutes. The cell pellet is suspended in 100µl PBS containing 1/1000 diluted primary antibody. The cell suspensions are incubated at 4°C for 30 minutes.
(4) Incubation of secondary antibody, FITC conjugate of Goat anti-mouse IgG, with tumor cells: the unbound primary antibodies are removed by discarding the supernatant after centrifuging at 1000g for 10 minutes. The cell pellet is suspended in 100µl PBS containing 1/1000 diluted secondary antibody. The cell suspensions are incubated at 4°C for 30 minutes.
(5) FCM analysis: the unbound secondary antibodies are removed by discarding the supernatant after centrifuging at 1000g for 10 minutes. The cell pellet is suspended in 400µl PBS and analyzed with FACS Calibur (BD). The extinction wavelength is 488nM. 10,000 cells are collected every time.

As shown in Fig.13, CEA-scTsAb binds to SW1116 and SK-OV-3 the best; CEA-scTsAb binds to A549 modestly; CEA-scTsAb does not bind to MCF-7.

### Example 7: FACSanalysis of the binding specificity of CEA-scTsAb to PBMC and Jurkat cells

A direct FCM method is used here to test the binding specificity of CEA-scTsAb to PBMC and Jurkat cells.
Operating:
(1) Conjugation of FITC (Sigma) to CEA-scTsAb: FITC is conjugated to CEA-scTsAb with the "Clark method" (Qiu Fa-zhu et al., 2002)
(2) Preparation of PBMC: PBMC are prepared by Ficoll gradient centrifugation (Qiu Fa-zhu et al., 2002) and cultured in 10% fetal calf serum (FCS)-containing RPMI1640 medium (Gibco) plus antibiotics (100 U penicillin) in a humidified 5 % CO₂ incubator at 37°C. After being incubated for 4 hours, the suspended cells, mainly lymphocytes, are transferred to a new flask. Thus the adherent cells are removed.
(3) Culture of Jurkat cells: Jurkat cells are cultured in 10% fetal calf serum (FCS)-containing RPMI1640 medium (Gibco) plus antibiotics (100 U penicillin) in a humidified 5 % CO2 incubator at 37°C.
(4) FACS analysis: 5×10⁵ PBMC or Jurkat cells in the exponential phase are collected by centrifuging at 1000g for 10 minutes and suspended in 100µl PBS, which contains 10µg/ml FITC conjugated of CEA-scTsAb. The cell suspensions are incubated at 4°C for 30 minutes then centrifuged at 1000g for 10 minutes. The cell pellet is re-suspended in 400µl PBS and analyzed with FACS Calibur (BD). The extinction wavelength is 488nM. 10,000 cells are collected every time.

As shown in Fig.14, CEA-scTsAb binds to PBMC and Jurkat cells specifically.

In summary of example 6 and example 7, CEA-scTsAb could bind to PBMC, Jurkat, and several tumor cells specifically, which is very important for inducing tumor specific cytolysis.

### Example 8: Detection of tumor specific cytolysis of colorectal carcinoma cell, SW1116, induced by CEA-scTsAb in the presence of lymphocytes.

In the system of an *in vitro* assay of tumor specific cytolysis, CEA expressing tumor cell line, SW1116, is used as the target cell (T), while lymphocytes from PBMC are used as the effector cells (E). After the target cells and effector cells are mixed together at a certain ratio of E/T, and CEA-scTsAb is added, tumor specific cytolysis is induced by incubating at 37°C for 48 hours. The result is then tested by MTT assay.
(1) PBMC are prepared according to example 7.
(2) SW1116 cells are cultured and collected according to example 6.
(3) SW1116 cells (1 × 10⁵/ml) are first plated in 96-well plates (Nunc) with 100µl/well. Then the effector cells (PBMC) are added at different E/T ratios (1, 5, 10) with 100µl/well. Concentrated CEA-scTsAbs are supplemented with 50µl/well to reach a final concentration of 1µg/ml. The mixture is incubated at 37°C 5%CO₂ incubator for about 48 hours. Quadruplicate wells are set for each concentration. The setting of negative controls: no CEA-scTsAb wells for each E/T ratio; the wells containing effector cells only; the wells containing target cells only; the wells containing no cells.
(4) MTT assay: the medium supernatants are removed by aspirating, and the adherent cells are washed with PBS one time. Add 200µl MTT solution (MTT: (3-(4, 5-dimethylthiazolyl-2)-2, 5-diphenyltetrazolium bromide, 500µg/ml, Sigma) for each well and incubate at 37°C for 4 hours. Wash the plate one time with PBS and add 200µl DMSO (Sigma) for each well. Continue to incubate at 37°C for 30 minutes. Absorbance of each well is measured at a wavelength of 570nm with background subtraction at 620nm.
(5) The percent of tumor specific cytolysis is calculated according to the formula:
   The percent of tumor specific cytolysis (%)=[A600 (ET)-A600 (ETA)]/[A600 (ET)-A600 (M)] × 100.
   A₆₀₀ (ET): the absorbance of the negative wells without CEA-scTsAb.
   A₆₀₀(ETA): the absorbance of the sample wells.
   A₆₀₀(M): the absorbance of the negative wells containing no cells.

The effect of E/T ratio on tumor specific cytolysis induced by CEA-scTsAb is shown in Fig.15. It can be concluded that E/T=5 is the optimal ratio, at which tumor specific cytolysis reaches 85%. It is suggested that there are other affecting factors for tumor specific cytolysis except E/T ratio. Fixing the E/T ratio at 5, the effect of increasing the concentration of CEA-scTsAb from 0.4 ng/ml to 12µg/ml on tumor specific cytolysis is shown in Fig.16. The curve displays four-stepwise phases for tumor specific cytolysis. In the first phase from 6µg/ml to 12µg/ml, the efficiency of tumor specific cytolysis displays negative correlation with the concentration of CEA-scTsAb and reaches a peak at 6 µg/ml; In the second phase from 750ng/ml to 6µg/ml it displayed a direct correlation and reaches the bottom at 750 ng/ml; In the third phase from 24ng/ml to 750ng/ml, it changes back into a negative correlation; In the fourth phase from 24ng/ml to zero, the direct correlation appeared again. Anyhow, two peaks of tumor specific cytolysis exist: 85% at 6 µg/ml and 70% at 24ng/ml. It can be concluded from the above data that extremely efficient tumor specific cytolysis could be induced at an even lower E/T ratio or lower concentration of CEA-scTsAb.

### Example 9: Morphological observation of tumor cells during the process of tumorspecific cytolysis.

After mixing PBMC (effector cells) with SW1116 cells (target cells) in L15 medium (10% FBS) at an E/T ratio of 5, and adding purified CEA-scTsAb at a concentration of 1 µg/ml, the mixture is incubated at 37°C for 20-40 h in a 5% CO₂ incubator. Then morphological changes of tumor cells and PBMC are observed under a 40× objective lens with an OLYMPUS IMT-2 inverted microscope, and recorded by photomicrography. As shown in Fig. 18 (A)-(G), there are four steps of morphological changes.

### Example 10: Detection of the proliferation of effector cells incubated with target cells and CEA-scTsAb.

The proliferation of effector cells (mainly T lymphocytes) detected with MTT assay can be used to evaluate the activation of T lymphocytes induced by co-incubated tumor cells and CEA-scTsAb. Operations:
(1) PBMC are prepared according to example 6.
(2) SW1116 tumor cells are cultured and collected according to example 6.
(3) Adjust the concentration of SW1116 cells to about 10⁶/ml in mitomycin C containing (25µg/ml Sigma) L15 medium and incubate the cell mixture at 37°C 5%CO₂-incubator for 20 minutes. After washing the tumor cells with PBS three times, residual mitomycin C is removed from culture medium.
(4) Adjust the concentration of SW1116 cells to 10⁵/ml and that of PBMC to 5X 10⁵/ml in L15 medium. Plate 100µl of the cells in a 96-well plate and incubate the cell mixture at 37°C in a 5% CO₂ incubator for 4 days. Quadruplicate wells are set for each concentration of CEA-scTsAb. Negative controls: no CEA-scTsAb wells for each E/T ratio; the wells containing effector cells only; the wells containing target cells only; the wells containing no cells.
(5) MTT assay: the medium supernatants are removed by aspirating, and the adherent cells are washed with PBS one time. Add 200µl MTT solution (MTT: (3-(4, 5-dimethylthiazolyl-2)-2, 5-diphenyltetrazolium bromide, 500µg/ml, Sigma) for each well and incubate at 37°C for 4 hours. Wash the plate one time with PBS and add 200µl DMSO (Sigma) to each well. Continue to incubate at 37°C for 30 minutes. Absorbance of each well is measured at a wavelength of 570nm with background subtraction at 620nm.
(6) The stimuli index (SI) is calculated according to the formula below:
   SI=[A₆₀₀ (ETA)-A600(ET)]
   A₆₀₀ (ET): the absorbance of the negative wells without CEA-scTsAb.
   A₆₀₀ (ETA): the absorbance of the sample wells.

As shown in Fig.17, There are three stepwise phases for stimuli index (SI). In the first phase from 750ng/ml to 12µg/ml, SI displays direct correlation with the concentration of CEA-scTsAb and reaches the bottom at 750ng/ml. In the second phase from 50ng/ml to 6µg/ml it displayed a negative correlation and reaches the peak at 50ng/ml. In the third phase from 50ng/ml to zero, it changes back into a direct correlation. It can be concluded that CEA-scTsAb possesses stimulating ability of T lymphocytes even at extremely lower concentrations. It is also found that tumor specific cytolysis induced by CEA-scTsAb corresponded to the activating state of co-incubated T lymphocytes. To sum up the results from examples 4-9, the function CEA-scTsAb focuses on two aspects: (1) retargeting effector cells around tumor cells; and (2) stimulating effector cells to kill target cells specifically. As described in Fig.19, retargeted cytotoxic T lymphocytes (CTL) are activated to kill target tumor cells directly; T helper cells secret cytokines, such as IL-2, IFN- γ and TNF- α, to assist CTL or natural killing cells (NK cell) in killing target tumor cells indirectly.

### Example 11. The mechanism of tumor specific cytolysis induced by CEA-scTsAb

There are three pathways for activated CTLs to kill tumor cells *in vivo(Kagi, Vignaux et al. 1994; Lowin, Hahne et al. 1994):* activated CTLs secret perforins to make holes on the membrane surface of tumor cells, which are broken up and induce necrosis; grazymes secreted by activated CTL can enter tumor cells through the above holes and induce apoptosis; activated CTL would be induced to express Fas ligands on its surface, which interact with Fas molecules on tumor cells and induce apoptosis. PI/annexin-V-FITC dual-color FCM (fluorescence cytometry) and subsequent fluorescence microphotography are used here to distinguish necrosis from apoptosis of tumor cells in an *in vitro* assay of tumor specific cytolysis.
(1) PBMC are prepared according to example 7.
(2) SW1116 cells are cultured and collected according to example 6.
(3) 10⁴ SW1116 cells in L15 medium are first plated in 48-well plate (Nunc) for each well. Then effector celis (PBMC) are added at an E/T ratio of 5. Concentrated CEA-scTsAb in L15 medium is supplemented to reach a final concentration of 1µg/ml. The mixture is incubated at 37°C 5%CO₂ incubator for about 20 hours. Quadruplicate wells are set for each concentration. Negative controls: no CEA-scTsAb wells for each E/T ratio; the wells containing effector cells only; the wells containing target cells only.
(4) Suspended effector cells and some dead tumor cells are collected by aspirating the medium from every well. The adherent cells are trypsinized and collected too. Combine these two cells in 1.5 ml vials.
(5) Being washed with PBS one time, the cell pellet of each well is prepared by centrifuging at 1000g for 10 minutes are then suspended in 100µl binding buffer (BD) and incubated with 5µl FITC conjugate of Annexin-V (BD) and 5µl PI solution (Sigma, 50µg/ml). The mixture is incubated at room temperature in the dark for 10 minutes.
(6) After being supplemented with 300µl binding buffer and photographed under a fluorescence microscope (Leica DMRA2), the results are shown in Fig. 20.
(7) The diluted cell mixtures are analyzed by FCM (FACS Calibur, BD) directly. The extinction wavelength is 488nM. 20,000 cells are collected for each vial.
   As shown in Fig.20, early apoptosis, late apoptosis, and necrosis are distinguished with two dyes: early apoptosis cells are dyed with green fluorescence (FITC conjugate of annexin V) only; necrosis cells are dyed with red fluorescence (PI) only; late apoptosis cells are dyed with both of them.
   In Fig.21, four quadrants represent four states of tumor cells: the upper left quadrant (UL) is necrosis cells; the upper right quadrant (UR) is late apoptosis cells; the lower left quadrant is live cells; the lower right quadrant is early apoptosis cells. The representative results are shown in Fig, 21. Negative controls without CEA-scTsAb: UL is 5.94%; UR is 2.23%; LL is 90.17%; LR is 1.66. Sample wells: UL is 9.80%; UR is 21.25%; LL is 52.83%; LR is 16.12%. It can be concluded that both types of tumor specific cytolysis are induced: necrosis and apoptosis. Compared with negative control, both early apoptosis and late apoptosis are increased 9 fold, while necrosis is increased 2 fold.

### Reference:

Atwell, J. L., K. A. Breheney, et al. (1999). "scFv multimers of the anti-neuraminidase antibody NC10: length of the linker between VH and VL domains dictates precisely the transition between diabodies and triabodies." Protein Eng 12(7): 597-604.
Ausubel, F. M. (1999). Short protocols in molecular biology : a compendium of methods from Current protocols in molecular biology. New York, Wiley.
Baxter, A. G. and P. D. Hodgkin (2002). "Activation rules: the two-signal theories of immune activation." Nat Rev Immunol 2(6): 439-46.
Bernard, A., Lamy, et al. (2002). "The two-signal model of T-cell activation after 30 years." Transplantation 73(1 Suppl): S31-5.
Cheng, J., X. Wang, et al. (2002). "Construction and expression of a reshaped VH domain against human CD28 molecules." Prep Biochem Biotechnol 32(3): 239-51.
Daniel, P. T., A. Kroidl, et al. (1998). "Immunotherapy of B-cell lymphoma with CD3x19 bispecific antibodies: costimulation via CD28 prevents "veto" apoptosis of antibody-targeted cytotoxic T cells." Blood 92(12): 4750-7.
Dolezal, O., L. A. Pearce, et al. (2000). "ScFv multimers of the anti-neuraminidase antibody NC10: shortening of the linker in single-chain Fv fragment assembled in V(L) to V(H) orientation drives the formation of dimers, trimers, tetramers and higher molecular mass multimers." Protein Eng 13(8): 565-74.
Dreier, T., P. A. Baeuerle, et al. (2003). "T cell costimulus-independent and very efficacious inhibition of tumor growth in mice bearing subcutaneous or leukemic human B cell lymphoma xenografts by a CD19-/CD3- bispecific single-chain antibody construct." J Immunol 170(8): 4397-402.
Dreier, T., G. Lorenczewski, et al. (2002). "Extremely potent, rapid and costimulation-independent cytotoxic T-cell response against lymphoma cells catalyzed by a single-chain bispecific antibody." Int J Cancer 100(6): 690-7.
Fan, H., C. Villegas, et al. (1998). "Myc-epitope tagged proteins detected with the 9E10 antibody in immunofluorescence and immunoprecipitation assays but not in western blot analysis." Biochem Cell Biol 76(1): 125-8.
Fang, M., R. Zhao, et al. (2004). "Characterization of an anti-human ovarian carcinomaxanti-human CD3 bispecific single-chain antibody with an albumin-original interlinker." Gynecol Oncol 92(1): 135-46.
Fang M, Z. R., Li H, Jiang X, Yin C, Lin Q, Huang H (2002). "Construction and expression of an anti-human ovarian carcinoma-anti-human CD3 bispecific single-chain antibody and its refolding studies." High Technol. Lett 12(11): 47-50.
Feil, G., H. W. Wechsel, et al. (1999). "Immunohistological investigations of carcinoembryonic antigen (CEA) in urothelial carcinomas." Anticancer Res 19(4A): 2591-7.
Foss, F. M. (2002). "Immunologic mechanisms of antitumor activity." Semin Oncol 29(3 Suppl 7): 5-11.
French, R. R. (1998). "Production of bispecific and trispecific F(ab)2 and F(ab)3 antibody derivatives." Methods Mol Biol 80: 121-34.
Ganjei, P., M. Nadji, et al. (1988). "Histologic markers in primary and metastatic tumors of the liver." Cancer 62(9): 1994-8.
Hengen, P. (1995). "Purification of His-Tag fusion proteins from Escherichia coli." Trends Biochem Sci 20(7): 285-6.
Holliger, P., O. Manzke, et al. (1999). "Carcinoembryonic antigen (CEA)-specific T-cell activation in colon carcinoma induced by anti-CD3 x anti-CEA bispecific diabodies and B7 x anti-CEA bispecific fusion proteins." Cancer Res 59(12): 2909-16.
Horie, Y., K. Miura, et al. (1996). "Marked elevation of plasma carcinoembryonic antigen and stomach carcinoma." Cancer 77(10): 1991-7.
Jung, G., M. Brandl, et al. (2001). "Local immunotherapy of glioma patients with a combination of 2 bispecific antibody fragments and resting autologous lymphocytes: evidence for in situ t-cell activation and therapeutic efficacy." Int J Cancer 91(2): 225-30.
Jung, G., U. Freimann, et al. (1991). "Target cell-induced T cell activation with bi- and trispecific antibody fragments." Eur J Immunol 21(10): 2431-5.
Kagi, D., F. Vignaux, et al. (1994). "Fas and perforin pathways as major mechanisms of T cell-mediated cytotoxicity." Science 265(5171): 528-30.
Kammerer, U., F. Thanner, et al. (2003). "Expression of tumor markers on breast and ovarian cancer cell lines." Anticancer Res 23(2A): 1051-5.
Kodama, H., M. Suzuki, et al. (2002). "Specific and effective targeting cancer immunotherapy with a combination of three bispecific antibodies." Immunol Lett 81(2): 99-106.
Koga, H., H. Kanda, et al. (1990). "Mouse-human chimeric monoclonal antibody to carcinoembryonic antigen (CEA): in vitro and in vivo activities." Hybridoma 9(1): 43-56.
Kortt, A. A., O. Dolezal, et al. (2001). "Dimeric and trimeric antibodies: high avidity scFvs for cancer targeting." Biomol Eng 18(3): 95-108.
Kuo, W. R., S. M. Tsai, et al. (1996). "Significance of tumour markers in nasopharyngeal carcinoma." J Otolaryngol 25(1): 32-6.
Li-ping, S., C. Ju-Iong, et al. (2003). "A new model of trispecific antibody resulting the cytotoxicity directed against tumor cells." Sheng Wu Hua Xue Yu Sheng Wu Wu Li Xue Bao (Shanghai) 35(6): 503-10.
Liu XF, X. S., Gu Z, Huang HL (1996). "Expression of anti-CD3 scFv and VH." Science in China (series C) 26(5): 428-435.
Loffler, A., M. Gruen, et al. (2003). "Efficient elimination of chronic lymphocytic leukaemia B cells by autologous T cells with a bispecific anti-CD19/anti-CD3 single-chain antibody construct." Leukemia 17(5): 900-9.
Loffler, A., P. Kufer, et al. (2000). "A recombinant bispecific single-chain antibody, CD19 x CD3, induces rapid and high lymphoma-directed cytotoxicity by unstimulated T lymphocytes." Blood 95(6): 2098-103.
Lowin, B., M. Hahne, et al. (1994). "Cytolytic T-cell cytotoxicity is mediated through perforin and Fas lytic pathways." Nature 370(6491): 650-2.
Manzke, O., H. Tesch, et al. (2001). "Locoregional treatment of low-grade B-cell lymphoma with CD3xCD19 bispecific antibodies and CD28 costimulation. I. Clinical phase I evaluation." Int J Cancer 91(4): 508-15.
Manzke, O., H. Tesch, et al. (2001). "Locoregional treatment of low-grade B-cell lymphoma with CD3xCD19 bispecific antibodies and CD28 costimulation. II. Assessment of cellular immune responses." Int J Cancer 91(4): 516-22.
Min Fang, X. J., Zhi Yang, Cong-Xiao Yu, Cheng-Chang Yin, Hua Li,Rui Zhao, Zhang-Zhang, Qin-Lin, Hua-Liang Huang (2003). "Effect of inter-linker on the activaty of single chain bispecific antibody." Chines science bulletin, 48(98): 1912-1918.
Nakamura, Y., T. Gojobori, et al. (2000). "Codon usage tabulated from international DNA sequence databases: status for the year 2000." Nucleic Acids Res 28(1): 292.
Sambrook, J. and D. W. Russell (2001). Molecular cloning: a laboratory manual. Cold Spring Harbor, N.Y., Cold Spring Harbor Laboratory Press.
Schoonjans, R., A. Willems, et al. (2000). "Efficient heterodimerization of recombinant bi- and trispecific antibodies." Bioseparation 9(3): 179-83.
Schoonjans, R., A. Willems, et al. (2000). "Fab chains as an efficient heterodimerization scaffold for the production of recombinant bispecific and trispecific antibody derivatives." J Immunol 165(12): 7050-7.
Schoonjans, R., A. Willems, et al. (2001). "A new model for intermediate molecular weight recombinant bispecific and trispecific antibodies by efficient heterodimerization of single chain variable domains through fusion to a Fab-chain." Biomol Eng 17(6): 193-202.
Shang-zhi, W., Y. Chang-cheng, et al. (2004). "Approach for Construction of a Completely Synthetic Human Antibody Library from Well-expressed Framework." High Technology Letters 14(6): 32-38.
Tuft, A., G. T. Stevenson, et al. (1991). "Trispecific F(ab')3 derivatives that use cooperative signaling via the TCR/CD3 complex and CD2 to activate and redirect resting cytotoxic T cells." J Immunol 147(1): 60-9.
Willems, A., J. Leoen, et al. (2003). "Optimizing expression and purification from cell culture medium of trispecific recombinant antibody derivatives." J Chromatogr B Analyt Technol Biomed Life Sci 786(1-2): 161-76.
Wong, W. M., S. A. Vakis, et al. (2000). "Rheumatoid arthritis T cells produce Th1 cytokines in response to stimulation with a novel trispecific antibody directed against CD2, CD3, and CD28." Scand J Rheumatol 29(5): 282-7.
Zhang, Z., Z. H. Li, et al. (2002). "Overexpression of DsbC and DsbG markedly improves soluble and functional expression of single-chain Fv antibodies in Escherichia coli." Protein Expr Purif 26(2): 218-28.

## Claims

1. A linear single chain recombinant trispecific antibody (scTsAb) constructed by fusing an anti-Carcinoma-Embryonic Antigen (CEA) single-chain antibody fragment of the variable region (scFv), an Fc linking peptide, an anti-CD3 single-chain antibody fragment of the variable region (scFv), a human serum albumin (HSA) linking peptide, and an anti-CD28 single-domain antibody (VH) in tandem.

2. The scTsAb as claimed in claim 1, wherein a c-myc-tag and/or a histidine-tag are fused to its C terminus.

3. The scTsAb as claimed in claim 1, wherein said anti-CEA scFv comprises the amino acid sequence of SEQ ID NO: 1.

4. The scTsAb as claimed in claim 1, wherein said anti-CD3 scFv comprises the amino acid sequence of SEQ ID NO: 2.

5. The scTsAb as claimed in claim 1, wherein said scTsAb comprises the amino acid sequence of SEQ ID NO: 4.

6. A DNA sequence encoding the scTsAb of claim 5, comprising the nucleic acid sequence of SEQ ID NO: 3.

7. An expression vector comprising the DNA sequence of claim 6.

8. The expression vector as claimed in claim 7, wherein said expressing vector is constructed by cloning the DNA sequence of an anti CEA single chain trispecific antibody (CEA-scTsAb) into pTRI.

9. A host cell comprising the expression vector of claim 7 or 8.

10. The host cell as claimed in claim 9, wherein said host cell is *E*. *coli.*

## Patentansprüche

1. Ein linearer, einzelkettiger, rekombinanter, trispezifischer Antikörper (scTsAb), hergestellt durch das Fusionieren eines einzelkettigen anti-Carcinoembryonales Antigen (CEA) Antikörperfragments der variablen Region (scFv), eines Fc verknüpfenden Peptids, eines einzelkettigen, anti-CD3 Antikörperfragments der variablen Region (scFv), eines humanes Serumalbumin (HSA)-verknüpfenden Peptids und eines anti-CD28 Einzeldomänenantikörpers (VH) hintereinander.

2. Der scTsAb wie in Anspruch 1 beansprucht, wobei ein c-Myc-Tag und/oder ein Histidin-Tag an seinen C Terminus fusioniert sind.

3. Der scTsAb wie in Anspruch 1 beansprucht, wobei das anti-CEA scFv die Aminosäuresequenz von SEQ ID NO: 1 umfasst.

4. Der scTsAb wie in Anspruch 1 beansprucht, wobei das anti-CD3 scFv die Aminosäuresequenz von SEQ ID NO: 2 umfasst.

5. Der scTsAb wie in Anspruch 1 beansprucht, wobei der scTsAb die Aminosäuresequenz von SEQ ID NO: 4 umfasst.

6. Eine DNS-Sequenz, die den scTsAb nach Anspruch 5 kodiert, umfassend die Nukleinsäuresequenz von SEQ ID NO: 3.

7. Ein Expressionsvektor, der die DNS-Sequenz nach Anspruch 6 umfasst.

8. Der Expressionsvektor, wie in Anspruch 7 beansprucht, wobei besagter Expressionsvektor hergestellt wird durch Klonierung der DNS-Sequenz von einem einzelkettigen anti-CEA trispezifischen Antikörper (CEA-scTsAb) in pTRI.

9. Eine Wirtszelle, die den Expressionsvektor nach Anspruch 7 oder 8 enthält.

10. Die Wirtszelle, wie in Anspruch 9 beansprucht, wobei die Wirtszelle *E*. *coli* ist

## Revendications

1. Anticorps trispécifique recombinant monocaténaire linéaire (scTsAb) construit par condensation d'un fragment d'anticorps monocaténaire anti-antigène carcino-embryonnaire (ACE) de la région variable (scFv), un peptide de liaison Fc, un fragment d'anticorps monocaténaire anti-CD3 de la région variable (scFv), un peptide de liaison à la sérumalbumine humaine (HSA), et un anticorps à un seul domaine anti-CD28 (VH) en tandem.

2. scTsAb selon la revendication 1, où une étiquette c-myc et/ou une étiquette histidine sont condensées à son extrémité C-terminale.

3. scTsAb selon la revendication 1, où ledit scFv anti-CEA comprend la séquence d'acides aminés de SEQ ID NO: 1.

4. scTsAb selon la revendication 1, où ledit scFv anti-CD3 comprend la séquence d'acides aminés de SEQ ID NO: 2.

5. scTsAb selon la revendication 1, ledit scTsAb comprenant la séquence d'acides aminés de SEQ ID NO: 4.

6. Séquence d'ADN codant pour le scTsAb selon la revendication 5, comprenant la séquence d'acides aminés de SEQ ID NO: 3.

7. Vecteur d'expression comprenant la séquence d'ADN selon la revendication 6.

8. Vecteur d'expression selon la revendication 7, ledit vecteur d'expression étant construit par clonage de la séquence d'ADN d'un anticorps trispécifique monocaténaire anti-CEA (CEA-scTsAb) en pTR1.

9. Cellule hôte comprenant le vecteur d'expression selon les revendications 7 ou 8.

10. Cellule hôte selon la revendication 9, ladite cellule hôte étant *E*. *coli.*
